# EUROPÄISCHE PATENTANMELDUNG

(11) **EP 3 578 106 A1**
(43) Veröffentlichungstag der Anmeldung: **11.12.2019**
(21) Anmeldenummer: 18175697.4
(22) Anmeldetag: 04.06.2018
(51) Int. Cl.: A61B 6/03, A61B 6/00, G01T 1/20, G02B 6/08

(54) **RÖNTGENDETEKTOR**

(71) Anmelder: Siemens Healthcare GmbH, 91052 Erlangen (DE)
(72) Erfinder: Tedde, Sandro Francesco, 91085 Weisendorf (DE); Düppenbecker, Peter Michael, 91074 Herzogenaurach (DE); Schmidt, Oliver, 91052 Erlangen (DE)

(57) **Zusammenfassung**

Röntgendetektor für eine Röntgeneinrichtung (1), wobei eine konvexe oder im Wesentlichen hohlzylindersegmentförmige Detektoroberfläche (8) des Röntgendetektors (6) gebildet wird, indem mehrere Halbleiterdetektoren (9, 10, 32, 39) genutzt werden, an deren der Detektoroberfläche (8) zugewandten Vorderseite (21) jeweils eine dem jeweiligen Halbleiterdetektor (9, 10) zugeordnete faseroptische Platte (12, 13) angeordnet ist, wobei die faseroptischen Platten (12, 13) jeweils wenigstens eine Seitenfläche (14, 15, 16, 17), die einen spitzen Winkel (18) mit der Vorderseite (11) des zugeordneten Halbleiterdetektors (9, 10) einschließt und die mit einer Seitenfläche (14, 15, 16, 17) einer weiteren der faseroptischen Platten (12, 13) verbunden ist, und/oder eine gekrümmte von dem Halbleiterdetektor (9, 10) abgewandte Vorderseite (21) aufweisen, oder indem wenigstens ein Halbleiterdetektor (32, 39) des Röntgendetektors (3) durch ein Substrat (33) getragen wird, dessen dem Halbleiterdetektor (32, 39) zugewandte Seite (34) und dessen von dem Halbleiterdetektor (32, 39) abgewandte Seite (35) gekrümmt sind.

## Beschreibung

Die Erfindung betrifft einen Röntgendetektor für eine Röntgeneinrichtung. Daneben betrifft die Erfindung eine Röntgeneinrichtung und ein Verfahren zur Herstellung eines Röntgendetektors.

Multimodale Bildgebungseinrichtungen gewinnen zunehmend an Bedeutung. In diesen wird ein Untersuchungsobjekt, beispielsweise der Körper eines Patienten, durch verschiedene Bildgebungsverfahren, beispielsweise durch ein röntgenbasiertes Verfahren, wie z. B. ein CT- oder ein Angiographieverfahren, und ein PET- oder Magnetresonanzverfahren abgebildet. Hierdurch können verschiedene einander ergänzende Informationen gewonnen werden. Hierbei ist es möglich, dass die verschiedenen Untersuchungsmodalitäten sequentiell durchgeführt werden, wobei beispielsweise ein Patiententisch für die unterschiedlichen Untersuchungsmodalitäten in unterschiedliche Positionen verfahren wird. Hierbei kann jedoch eine Bewegung des Patienten bzw. ein Fehler in der Registrierung resultieren. Daher ist es vorteilhaft, die verschiedenen Untersuchungsmodalitäten derart zu integrieren, dass sie gleichzeitig oder zumindest ohne eine Bewegung des Untersuchungsobjekts durchgeführt werden können.

Eine Integration eines Röntgendetektors in einen Magnetresonanztomographen ist beispielsweise aus den Druckschriften DE 10 2016 218 889 A1 und DE 10 2016 215 460 A1 bekannt. Sollen übliche Röntgendetektoren in ein System zur Magnetresonanzbildgebung integriert werden, resultieren jedoch erhebliche Bauraumprobleme. Dies kann beispielsweise dazu führen, dass ein Durchmesser eines Hauptmagneten erheblich vergrößert werden muss und/oder dass nur eine Röntgenerfassung aus bestimmten Perspektiven möglich ist.

Für Computertomographieanwendungen sind verschiedene Ansätze bekannt, gekrümmte Röntgendetektoren bereitzustellen, die potentiell beispielsweise in Magnetresonanzröhren genutzt werden könnten. Aufgrund der relativ großen Bildpunktgröße von ungefähr 1 mm² und dem Bildpunktabstand von ungefähr 100 µm können solche Detektoren relativ einfach dadurch gebildet werden, dass ebene Röntgendetektoren um eine Röhre herum angeordnet werden.

Alternativ hierzu ist es bekannt, biegbare Detektorflächen zu nutzen. Möglichkeiten hierfür sind beispielsweise aus dem Artikel von A. Nathan et al., "Amorphous silicon technology for large area digital X-ray and optical imaging", Microelectronics Reliability 42 (2002), S. 735 bzw. von der Internetseite "http://www.osadirect.com/news/article/2053/holst-centreimec-and-philips-demo-worlds-first-curved-plasticphotodetector/" bekannt. Mit den genannten Ansätzen werden bislang jedoch beispielsweise für Angiographieanwendungen keine ausreichenden Bildauflösungen und Abbildungsqualitäten erreicht.

Im Bereich kleinformatiger CMOS-Detektoren für Kameras ist es zudem bekannt, eine Substratdicke zu reduzieren, um einen biegbaren Sensor zu erhalten. Dies wird beispielsweise auf den Internetseiten http: "www.electronicsweekly.com/news/research-news/curved-image-sensor-simplifies-stream-optics-2018-02/" und "http://www.direporter.com/industry-news/awards-honors/sonyreveals-curved-cmos-image-sensors" erläutert. Die so hergestellten Detektoren weisen üblicherweise jedoch nur eine Größe von einigen cm² auf und werden an flachen Halterungen angeordnet. Großflächige Sensoren können durch diesen Ansatz nicht ohne weiteres hergestellt werden.

Der Erfindung liegt somit die Aufgabe zugrunde, einen Röntgendetektor anzugeben, der auch zur Aufnahme von hochauflösenden Röntgenaufnahmen, beispielsweise Angiographieaufnahmen, geeignet ist und auch bei gekrümmten Detektoranordnungen relativ kleinbauend ist, beispielsweise um eine Integration in Magnetresonanz- oder PET-Systeme zu ermöglichen.

Die Aufgabe wird erfindungsgemäß durch einen Röntgendetektor für eine Röntgeneinrichtung gelöst, wobei eine konvexe oder im Wesentlichen hohlzylindersegmentförmige Detektoroberfläche des Röntgendetektors gebildet wird, indem mehrere Halbleiterdetektoren genutzt werden, an deren der Detektoroberfläche zugewandten Vorderseite jeweils eine dem jeweiligen Halbleiterdetektor zugeordnete faseroptische Platte angeordnet ist, wobei die faseroptischen Platten jeweils wenigstens eine Seitenfläche, die einen spitzen Winkel mit der Vorderseite des zugeordneten Halbleiterdetektors einschließt und die mit einer Seitenfläche einer weiteren der faseroptischen Platten verbunden ist, und/oder eine gekrümmte von dem Halbleiterdetektor abgewandte Vorderseite aufweisen, oder indem wenigstens ein Halbleiterdetektor des Röntgendetektors durch ein Substrat getragen wird, dessen dem Halbleiterdetektor zugewandte Seite und dessen von dem Halbleiterdetektor abgewandte Seite gekrümmt sind.

Der Erfindung liegt die Idee zugrunde, zusätzliche, vorgeformte Elemente zu nutzen, die den Halbleiterdetektor oder die Halbleiterdetektoren tragen, also die faseroptischen Platten bzw. das Substrat. Hierdurch kann die relative Position und Orientierung der Halbleiterdetektoren zueinander bzw. die Krümmung bzw. Form eines biegbaren Halbleiterdetektors mit hoher Genauigkeit vorgegeben werden. Hierdurch kann ein großflächiger, dünner, konkaver bzw. hohlzylindersegmentförmiger Röntgendetektor aufgebaut werden, wobei durch die definierte Anordnung der Halbleiterdetektoren zueinander bzw. die genaue Vorgabe der Form eine hohe Messgenauigkeit und eine hohe Auflösung, insbesondere mit geringen Fehlern in Bereichen, in denen die Erfassungsbereiche von Halbleiterdetektoren aneinandergrenzen bzw. überlappen, erreicht werden. Hierdurch kann der erfindungsgemäße Röntgendetektor in Kombinationsgeräten, beispielsweise Magnetresonanztomographen, auch für hochauflösende Röntgenaufnahmen, beispielsweise für Angiographieaufnahmen, genutzt werden. Die Nutzung von dünnen, gekrümmten und hochauflösenden Röntgendetektoren ist jedoch auch in anderen Anwendungsbereichen zweckmäßig, so dass der erfindungsgemäße Röntgendetektor beispielsweise auch in reinen Röntgengeräten oder anderen Kombigeräten genutzt werden kann.

Der Röntgendetektor umfasst insbesondere wenigstens einen Szintillator, wobei der Halbleiterdetektor oder die Halbleiterdetektoren zur ortsaufgelösten Erfassung von durch den Szintillator bei einer Einstrahlung von Röntgenstrahlung abgegebener elektromagnetischer Strahlung dient bzw. dienen. Bei Verwendung mehrerer Halbleiterdetektoren kann jedem Szintillator ein Halbleiterdetektor zugeordnet sein, der dessen Licht erfasst oder das Licht eines jeweiligen Szintillators kann durch mehrere Halbleiterdetektoren erfasst werden. Die Halbleiterdetektoren sind insbesondere entlang einer Kreisbahn bzw. eines Kreissegments angeordnet. Die einzelnen Halbleiterdetektoren können hierbei gekrümmt sein, um der Kreis- bzw. Kreissegmentform zu folgen oder sie können jeweils eben und gewinkelt zueinander angeordnet sein. Werden beispielsweise flache Halbleiterdetektoren genutzt, die an einer jeweiligen faseroptischen Platte angeordnet sind, wobei die Seitenflächen, an denen die faseroptischen Platten verbunden sind, mit der Vorderseite des zugeordneten Halbleiterdetektors jeweils einen Winkel von 90°-α einschließen, so resultiert ein Winkel von 2α zwischen benachbarten Halbleiterdetektoren. Durch die Nutzung von faseroptischen Platten mit gekrümmter Vorderseite kann mit geringem technischen Aufwand eine definierte Krümmung der Detektoroberfläche bereitgestellt werden, insbesondere indem ein Szintillator mit konstanter Dicke auf diese Vorderseite aufgebracht, z.B. als dünne Folie aufgeklebt, wird. Die Krümmung der Vorderseite bzw. die Schrägstellung der Seitenwände kann z.B. durch ein Fräsen oder ein anderweitiges spanabhebendes Bearbeiten einer quaderförmigen faseroptischen Platte erzeugt werden.

Wird ein den wenigstens einen Halbleiterdetektor tragendes Substrat genutzt, so kann der Halbleiterdetektor insbesondere an der konkaven Seite des Substrats angeordnet sein. Der Szintillator kann unmittelbar auf der von dem Substrat abgewandten Seite des Halbleiterdetektors angeordnet sein. Alternativ ist es jedoch auch möglich, eine Zwischenschicht, beispielsweise eine faseroptische Platte, zwischen dem Halbleiterdetektor und dem Szintillator anzuordnen. In jenem Fall, in dem die Vorderseite des jeweiligen Halbleiterdetektors an der faseroptischen Platte angeordnet ist, wird der Szintillator vorzugsweise auf die von dem Halbleiterdetektor abgewandte Oberfläche dieser faseroptischen Platte aufgebracht. Wie später noch detailliert erläutert werden wird, kann durch eine Krümmung dieser Oberfläche der faseroptischen Platte erreicht werden, dass der Szintillator auch bei einer gekrümmten Detektoroberfläche eine im Wesentlichen konstante Dicke aufweist.

Der Krümmungsradius der dem Halbleiterdetektor zugewandten Seite des Substrats kann zumindest lokal kleiner als 2 m, insbesondere kleiner als 1 m sein. Er ist insbesondere so gewählt, dass die Detektoroberfläche, also insbesondere die Szintillatoroberfläche, einen Krümmungsradius zwischen 35 cm und 45 cm, insbesondere zwischen 37 cm und 43 cm oder zwischen 39 cm und 41 cm aufweist.

Wenigstens eine der faseroptischen Platten kann wenigstens zwei, wenigstens drei oder wenigstens vier Seitenflächen aufweisen, die in spitzem Winkel zu der Vorderseite des zugeordneten Halbleiterdetektors stehen und die mit einer Seitenfläche einer jeweiligen weiteren der faseroptischen Platten verbunden sind. Sind zwei dieser zur Verbindung genutzten Seitenflächen an gegenüberliegenden Seiten der faseroptischen Platten angeordnet, können hier durch längere Kreisbögen oder Ähnliches durch mehrere faseroptische Platten mit jeweils daran angeordneten Halbleiterdetektoren zusammengesetzt werden. Drei bzw. vier zur Verbindung genutzte Seitenflächen können genutzt werden, um eine flächige Anordnung der faseroptischen Platten und somit der Halbleiterdetektoren zu realisieren. In einer besonders bevorzugten Variante weisen nur zwei der Seitenflächen einer jeweiligen faseroptischen Platte einen spitzen Winkel zu der Vorderseite des zugeordneten Halbleiterdetektors auf und die beiden anderen, ebenfalls zur Verbindung genutzten Seitenflächen stehen senkrecht auf dem zugeordneten Halbleiterdetektor. Somit wird eine Detektoroberfläche realisiert, die nur in einer Richtung gekrümmt ist und die beispielsweise die Form eines Hohlzylinders bzw. eines Hohlzylindersegments aufweisen kann.

Als Halbleiterdetektor wird vorzugsweise ein CMOS-Detektor genutzt. Die Größe eines einzelnen Bildpunkts kann zwischen 30 µm und 300 µm sein. Der Abstand zwischen den Bildpunkten kann kleiner als 50 µm sein. Daher ist bei einem Zusammensetzen einer Detektoroberfläche hohe Genauigkeit erforderlich, wenn keine Messdaten an Grenzflächen zwischen verschiedenen Modulen bzw. Halbleiterdetektoren verloren gehen sollten. Vorzugsweise ist an der Grenze der Abbildungsbereiche zweier Halbleiterdetektoren der Abstand der erfassbaren Bildpunkte kleiner oder gleich drei oder kleiner oder gleich zwei oder kleiner oder gleich einer Bildpunktbreite. Werden mehrere faseroptische Platten genutzt, kann ergänzend oder alternativ der Abstand von benachbarten Fasern verschiedener faseroptischer Platten an der Grenze der Vorderseiten dieser Platten kleiner oder gleich drei oder kleiner oder gleich zwei oder kleiner oder gleich einer Bildpunktbreite sein.

Die einzelnen Halbleiterdetektoren können ungefähr 1 mm dick sein. Insbesondere sind sie zwischen 300 µm und 1,5 mm dick, wobei Dicken von weniger als 1 mm oder weniger als 700 µm bevorzugt sind. Soll eine Anordnung auf einem gekrümmten Substrat erfolgen, können vorzugsweise Dicken von weniger als 500 µm, weniger als 400 µm oder weniger als 300 µm genutzt werden, um die vorangehend erwähnten Krümmungsradien zu erreichen.

Bei einem Aufbau des Röntgendetektors mit mehreren faseroptischen Platten kann die Seitenlänge der dem Halbleiterdetektor zugewandten Rückseite einer jeweiligen faseroptischen Platte zwischen einigen cm und einigen 10 cm sein. Insbesondere ist diese Seitenlänge zwischen 1 cm und 20 cm, wobei Ausdehnungen von weniger als 15 cm bzw. weniger als 10 cm bevorzugt werden. Dies ermöglicht eine Zusammensetzung des Röntgendetektors aus relativ kleinen Modulen, die jeweils eine faseroptische Platte und zumindest einen zugeordneten Halbleiterdetektor umfassen. Dies hat den Vorteil, dass kleinflächige Halbleiterdetektoren einfach und günstiger herstellbar sind und dass bei gleichem Krümmungsradius des Gesamtdetektors der Winkel zwischen benachbarten Halbleiterdetektoren kleiner sein kann, womit potentielle Artefakte bei der Bildgebung gering gehalten werden können und eine sehr geringe Bautiefe erreicht werden kann.

Die faseroptischen Platten können eine Dicke zwischen einigen mm und einigen cm aufweisen. Beispielsweise kann eine Dicke von zwischen 1 mm und 50 mm genutzt werden. Vorzugsweise ist die Dicke kleiner als 10 mm bzw. kleiner als 7 mm. Beispielsweise kann die Dicke in diesen Fällen zwischen 0,5 mm und 10 mm oder zwischen 0,7 mm und 7 mm liegen. Es kann eine faseroptische Platte genutzt werden, die Glasfasern oder aus einem Polymer gebildete Fasern umfasst. Die einzelnen Fasern können einige µm bis einige 100 µm dick sein. Die Dicke der Fasern kann zwischen 1 µm und 500 µm liegen, wobei sie vorzugsweise kleiner als 100 µm oder kleiner als 10 µm ist.

Die faseroptische Platte kann mit dem Halbleiterdetektor verklebt sein. Hierzu kann ein durchsichtiges beidseitiges Klebeband oder ein durchsichtiger Klebstoff, beispielsweise Epoxidharz oder ein anderes Harz oder Acrylatkleber, verwendet werden. Bevorzugt ist das Verbindungsmaterial so gewählt, dass die Lichteinkopplung von der faseroptischen Platte in den Halbleiterdetektor optimiert wird. Dies kann beispielsweise dadurch erreicht werden, dass der Brechungsindex des Verbindungsmaterials n_{V} etwa der Wurzel des Produkts aus dem Brechungsindex der faseroptischen Platte n_{F} und dem Brechungsindex des Halbleiterdetektors n_{D} entspricht. Die faseroptische Platte weist insbesondere eine ebene Rückseite auf, an der der Halbleiterdetektor angeordnet ist. Hierdurch wird insbesondere erreicht, dass ebene Halbleiterdetektoren genutzt werden können. Die von dem Halbleiterdetektor abgewandte Vorderseite der faseroptischen Platte kann eben sein, womit die faseroptische Platte besonders einfach herstellbar ist. Besonders bevorzugt ist sie jedoch gekrümmt, um eine einfache Aufbringung des Szintillators zu ermöglichen. Insbesondere ermöglicht dies eine Nutzung eines Szintillators mit konstanter Dicke, auch wenn insgesamt eine gekrümmte Oberfläche erreicht werden soll.

In dem erfindungsgemäßen Röntgendetektor ist es möglich, dass mehrere Halbleiterdetektoren gemeinsam zur Bildgebung genutzt werden können. In Bildbereichen, in denen Abbildungsbereiche überlappen bzw. aneinandergrenzen, kann es trotz der erfindungsgemäß ermöglichten hochgenauen Anordnung möglich sein, dass dort Verzerrungen und/oder Bilddefekte auftreten. Bei einer Nutzung einer faseroptischen Platte kann es zudem aufgrund einer Wechselwirkung von benachbarten Fasern zu einer Beeinflussung deren für einzelne Bildpunkte erfassten Röntgenintensitäten durch tatsächlichen an benachbarten Bildpunkten vorhandene Röntgenintensitäten kommen. Es kann daher vorteilhaft sein, den erfindungsgemäßen Röntgendetektor gemeinsam mit einer Verarbeitungseinrichtung oder einem Softwareprodukt zu nutzen, das entsprechende Bildfehler zumindest weitgehend korrigieren kann. Dies kann beispielsweise dadurch erfolgen, dass Abbildungsfehler durch Referenzaufnahmen detektiert werden und so erkannte Fehler korrigiert werden können.

Die dem Halbleiterdetektor zugewandte Rückseite der faseroptischen Platte kann jeweils eben sein. Wie erläutert kann zudem die Vorderseite gekrümmt sein. Der Krümmungsradius der Vorderseite kann zumindest lokal kleiner als 2 m oder 1 m sein. Vorzugsweise ist der Krümmungsradius zwischen 35 cm und 45 cm, insbesondere wenn der Röntgendetektor in eine Röhre eines Magnetresonanztomographen integriert werden soll. Die Kombination einer ebenen Rückseite und einer gekrümmten Vorderseite ermöglicht es, ebene Halbleiterdetektoren und Szintillatorschichten mit konstanter Dicke zur Bildung gekrümmter Detektoroberflächen zu nutzen.

Wenigstens eine Seitenfläche wenigstens einer der faseroptischen Platten kann senkrecht auf der Vorderseite dieser faseroptischen Platte stehen. Dies kann insbesondere die Seitenfläche oder eine der Seitenflächen sein, an der die faseroptische Platte mit einer weiteren der faseroptischen Platten verbunden ist. Ergänzend oder alternativ kann die Vorderseite der faseroptischen Platte in wenigstens einer senkrecht auf der Vorderseite des Halbleiterdetektors stehenden Schnittebene eine Kreissegmentform aufweisen, wobei sich die Seitenfläche in Radialrichtung dieses Kreissegments erstreckt. Weist die Vorderseite zumindest in der Schnittebene eine Kreissegmentform auf, so bedeutet dies, dass sie zumindest in der Schnittebene einen im Wesentlichen konstanten Krümmungsradius aufweist. Vorzugsweise ist der Krümmungsradius in allen zu dieser ersten Schnittebene parallelen Schichtebenen ebenfalls konstant und vorzugsweise gleich zum Krümmungsradius in der ersten Schnittebene. Die Vorderseite kann somit die Form eines Segments einer Zylinderwandsegments aufweisen. Selbstverständlich kann die Form der Vorderseite, beispielsweise aufgrund von produktionsbedingten Toleranzen, geringfügig von der Kreissegmentform bzw. der Form des Zylinderwandsegments abweichen. Vorzugsweise kann an zwei gegenüberliegenden Seiten der Vorderseite jeweils eine Seitenwand der faseroptischen Platte anschließen, die sich in die Radialrichtung erstreckt. Durch die Stellung der Seitenfläche senkrecht zur Vorderseite bzw. in Radialrichtung wird erreicht, dass die an dieser Seitenfläche zusammengesetzten faseroptischen Platten durch ihre Vorseiten eine gemeinsame glatte Kurve, insbesondere mit einer Kreis- bzw. Kreissegmentform, bilden. Dies ermöglicht einen einfachen Aufbau relativ großer gekrümmter, insbesondere hohlzylinderförmiger bzw. hohlzylindersegmentförmiger, Röntgendetektoren.

Wenigstens eine der faseroptischen Platten kann die Vorderseite des zugeordneten Halbleiterdetektors oder dessen fotoaktive Fläche zumindest in eine Richtung vollständig bedecken oder zumindest an einer oder zumindest an zwei Seiten über den Halbleiterdetektor oder dessen fotoaktive Fläche hinausragen. Anders ausgedrückt kann die von dem zugeordneten Halbleiterdetektor abgewandte Rückseite der faseroptischen Platte zumindest in eine Richtung die gleiche Größe aufweisen wie die Vorderseite des zugeordneten Halbleiterdetektors. Ein Halbleiterdetektor, der die gleiche Größe wie die Rückseite der faseroptischen Platte aufweist, ist vorteilhaft, da das gesamte durch die Fasern der faseroptischen Platte transportierte Licht in diesem Fall durch den Halbleiterdetektor erfasst werden kann. Dies kann beispielsweise dazu dienen, sehr hohe Auflösungen zu erreichen, wenn beispielsweise die Rückseite der faseroptischen Platte größer ist als die Vorderseite und entsprechend ortsabhängig schrägstehende Fasern genutzt werden, um ein auf die Vorderseite der faseroptischen Platte projiziertes Bild an der Rückseite vergrößert auf den zugeordneten Halbleiterdetektor zu projizieren. Bei einer zylindersegmentförmigen Detektorfläche kann die Vergrößerung bzw. Stauchung insbesondere in Umfangsrichtung erfolgen. Die beschriebene bauartbedingte Ver- bzw. Entzerrung kann durch eine softwareseitige Nachverarbeitung ergänzt werden, die insbesondere die Bilddaten in verschiedene Richtungen verschieden stark verszerren kann.

Um einen einfachen Aufbau des Röntgendetektors zu ermöglichen, können auch faseroptische Platten genutzt werden, deren Fasern jeweils parallel zueinander sind und insbesondere senkrecht auf der Vorderseite des zugeordneten Halbleiterdetektors stehen. Wird ausschließlich die Vorderseite einer solchen faseroptischen Platte durch einen Szintillator bedeckt, wird in Fasern, die nicht an die Vorderseite grenzen, durch den Szintillator bei Annahme einer idealen Faser kein Licht eingekoppelt. Wird in diesem Fall ein Halbleiterdetektor genutzt, dessen Größen im Wesentlichen der Größe der Vorderseite entspricht, wird, obwohl die faseroptische Platte über den Halbleiterdetektor seitlich hinausragt, im Wesentlichen kein Licht, das durch den Szintillator in die faseroptische Platte eingekoppelt wurde, an dem Halbleiterdetektor verbeigeführt. Somit gehen trotz Nutzung eines kleineren Halbleiterdetektors keine Messinformationen verloren.

Es kann jedoch vorteilhaft sein, größere Halbleiterdetektoren zu nutzen, um auch Licht zu erfassen, dass durch Abweichungen vom Idealverhalten in Fasern eingekoppelt wurde, die nicht an der Vorderseite enden. Dies kann dazu dienen, die Abweichung von dem Idealverhalten zu quantifizieren und diese Information im Rahmen einer Nachbearbeitung der Daten zur Reduzierung von Messartefakten bzw. zur Qualitätsverbesserung zu nutzen. Beispielsweise kann mit Hilfe von Intensitäten, die für Fasern erfasst wurden, in die im Idealfall kein Licht eingekoppelt würde, ein näherungsweiser Faltungs-Kernel berechnet werden, der die Bildfehler bzw. Bildpunktwechselwirkungen aufgrund eines Lichtübertritts zwischen Fasern beschreibt. Dieser kann genutzt werden, um eine näherungsweise Entfaltung durchzuführen und somit insbesondere den Bildkontrast zu verbessern.

In einigen Fällen kann es, insbesondere aus Kostengründen, auch gewünscht sein, noch kleinere Halbleiterdetektoren zu nutzen. Um dennoch einen Verlust von Messdaten zu vermeiden, können die Orientierungen der Fasern in der faseroptischen Platte so gewählt werden, dass ein auf die Vorderseite projiziertes Bild an der Rückseite auf die Fläche des Halbleiterdetektors verkleinert wird.

Die Fasern der jeweiligen faseroptischen Platte können senkrecht zu der Vorderseite des zugeordneten Halbleiterdetektors stehen oder ein Winkel zwischen den Fasern der faseroptischen Platte und der Vorderseite des Halbleiterdetektors kann in wenigstens einer Richtung monoton mit der Position des halbleiterdetektorseitigen Endes der Fasern variieren. Werden Fasern genutzt, die senkrecht zu der Vorderseite des zugeordneten Halbleiterdetektors stehen, wird ein auf die Vorderseite projiziertes Bild im Wesentlichen unverändert auf den Halbleiterdetektor projiziert. Durch eine monotone Variation des Winkels mit der Position des halbleiterdetektorseitigen Endes der Faser kann insbesondere eine Streckung oder Stauchung des projizierten Bildes in die jeweilige Richtung erfolgen. Dies kann genutzt werden, um höhere Auflösungen zu realisieren oder um kleinere Halbleiterdetektoren nutzen zu können, was insbesondere zur Kostenersparnis dienen kann. Weisen beispielsweise die Rückseite der faseroptischen Platte und der Halbleiterdetektor die gleiche Ausdehnung auf, so können die Fasern im Bereich der spitzwinkeligen Seitenflächen insbesondere im Wesentlichen parallel zu diesen Seitenflächen verlaufen und die Fasern im Mittenbereich des Halbleiterdetektors können im Wesentlichen senkrecht auf diesem stehen.

Die von dem Halbleiterdetektor abgewandte Vorderseite der faseroptischen Platte kann, wie vorangehend erläutert, in wenigstens einer senkrecht auf der Vorderseite des Halbleiterdetektors stehenden Schnittebene eine Kreissegmentform aufweisen. In diesem Fall können die in dieser Schnittebene liegenden Fasern der faseroptischen Platte sich jeweils in Radialrichtung dieses Kreissegments erstrecken. Dies führt dazu, dass die einzelnen Fasern und somit auch Detektoren, die das Licht der Fasern erfassen, gleichmäßig Winkeln in der Schnittebene zugeordnet werden, womit Artefakte bzw. Verzerrungen einer Röntgenaufnahme durch den Röntgendetektor vermieden oder zumindest deutlich verringert werden können. Eine derartige Anordnung ist besonders Vorteilhaft, wenn ein Fokuspunkt einer Röntgenquelle in dem Mittelpunkt dieses Kreises liegt. Dies kann insbesondere bei reinen Röntgeneinrichtungen realisiert werden. Wird eine kombinierte Bildgebungseinrichtung, beispielsweise zur kombinierten Magnetresonanz-und Röntgenbildgebung genutzt, so kann es stattdessen Vorteilhaft sein, wenn die Kontur der Detektorfläche ein Zylinderwandsegment um ein Isozentrum der zweiten Bildgebungsmodalität herum bildet. Die Ausrichtung der Fasern kann in diesem Fall jedoch vorteilhaft so gewählt werden, dass sie weiterhin auf einen Fokuspunkt einer Röntgenquelle hin ausgerichtet sind, der insbesondere weiter von der Detektoroberfläche entfernt sein kann als das Isozentrum und insbesondere außerhalb einer Röhre zur Aufnahme des Untersuchungsobjekts angeordnet sein kann. Die Fasern stehen somit insbesondere weder senkrecht auf der Vorderseite des Halbleiterdetektors noch senkrecht auf der Detektoroberfläche bzw. der Vorderseite der faseroptischen Platte.

Eine besonders bevorzugte Ausgestaltungsform der Erfindung sieht die Integration des Röntgendetektors in einem Magnetresonanztomographen vor. In diesem Fall kann der Röntgendetektor eine zylindrische Form aufweisen, wobei der Zylinder einen Radius von 35-45 cm besitzt. Des Weiteren kann in dem gewählten Ausführungsbeispiel eine Röntgenröhre außerhalb des Magnetresonanztomographen angeordnet sein und der Abstand zum Detektor kann typischerweise 1-2 m betragen. Es ist verständlich, dass in diesem Fall das Isozentrum des Magnetresonanztomographen, welches die Kreissegmentform des Detektors vorgeben kann, nicht mit dem Kreisbogen übereinstimmt, der durch einen Kreis um die Röntgenquelle bzw. um deren Fokuspunkt als Mittelpunkt und dem Abstand zwischen Röntgenquelle und Röntgendetektor als Radius, beschrieben wird.

Dieser Symmetriebruch führt zu Bildverzerrungen, die durch eine Computereinrichtung korrigiert werden können. Dabei muss berücksichtigt werden, dass durch die zylinderförmige Ausgestaltung des Detektors, die Symmetrie in axiale Richtung des Zylinders und in tangentiale Richtung des Zylinders verschieden ist. So ist der Detektor in tangentialer Richtung stärker gekrümmt als die von der Röntgenquelle auslaufende Kugelwelle, was unter Annahme eines idealen gekrümmten Detektors ohne weitere Effekte der faseroptischen Platte zu einer Dehnung des Bildes an den Randbereichen führt. Dagegen weist der Detektor in axialer Richtung eine geringere Krümmung auf als die von der Röntgenquelle auslaufende Kugelwelle bzw. keine Krümmung, was zu ebenfalls zu einer Bilddehnung im Randbereich des Detektors führt. Im Allgemeinen ist jedoch davon auszugehen, dass die Dehnung in die beiden Symmetrierichtungen unterschiedlich stark ist und in diesem Ausführungsbeispiel also eine Korrektur des Röntgenbildes benötigt wird, welche in axialer Richtung eine Stauchung des Bildes ausführt und in tangentialer Richtung eine Stauchung, die stärker ausfällt als in axialer Richtung.

Durch die faseroptische Platte kann die oben genannte Stauchung in tangentialer Richtung verstärkt oder reduziert werden. Bevorzugt ist die faseroptische Platte dabei so ausgelegt, dass die Verzerrung aus oben genannten Geometriegründen teilweise oder ganz kompensiert wird.

Auf die von dem Halbleiterdetektor abgewandte Vorderseite der jeweiligen faseroptischen Platten oder auf eine durch die Vorderseiten von mehreren über ihre jeweiligen Seitenflächen verbundenen faseroptischen Platten gebildete Gesamtvorderseite kann ein Szintillator aufgebracht sein. Da viele Szintillatormaterialien hygroskopisch sind, kann es erforderlich sein, den Szintillator zu versiegeln, beispielsweise durch eine Lack- oder Harzschicht. Dies ist besonders einfach möglich, wenn der Szintillator erst nach dem Zusammensetzen der faseroptischen Platten auf deren Gesamtvorderseite aufgebracht wird, da die offenliegende Oberfläche minimiert wird. In einigen Fällen kann es jedoch auch vorteilhaft sein, den Szintillator jeweils auf die einzelnen faseroptischen Platten aufzubringen und diese erst anschließend zusammenzusetzen bzw. nach dem Zusammensetzen separate Szintillatoren für die einzelnen Platten vorzusehen. Der Szintillator kann beispielsweise direkt oder mit Hilfe einer Binderlage auf die faseroptische Platte aufgebracht werden. Alternativ ist es auch möglich, den Szintillator auf ein transparentes Substrat aufzubringen und dieses auf die faseroptische Platte oder die Gesamtvorderseite aufzukleben. Die Dicke des Szintillators kann zwischen 100 µm und 3000 µm, insbesondere zwischen 100 µm und 2000 µm, speziell zwischen 200 µm und 1000 µm, sein.

Als Szintillatormaterial kann CsI:Tl oder Gd₂O₂S:Pr,Ce oder Gd₂O₂S:Tb oder Gd₂O₂S:Pr,Ce,F oder YAG:Ce oder CdI₂:Eu oder Lu₂O₃:Tb oder Lu₂SiO₅:Ce oder Lu_{1.8}Y_{0.2}SiO₅:Ce oder CdWO₄ oder CsI:Na oder NaI:Tl oder Bi₄Ge₃O₁₂ oder Gd₂SiO₅ oder CsBr:Eu oder Lu₂O₃:Eu oder Lu₂O₃:Tb oder Gd₂O₃:Eu oder YGdO: (Eu,Pr) oder GdGaO:Cr,Ce oder CuI oder ein beliebiges anderes organisches oder anorganisches Szintillatormaterial mit einem insbesondere auf den Halbleiterdetektor abgestimmten Absorptionsspektrum verwendet werden. Die vor dem jeweiligen Doppelpunkt stehenden Elementen geben hierbei die Grundstruktur des Szintillatormaterials an und nach dem Doppelpunkt genannte Elemente dienen zur Dotierung. Werden mehrere Elemente in Klammern genannt, sind diese alternativ zur Dotierung nutzbar.

Der oder ein auf die faseroptische Platte oder auf die Gesamtvorderseite oder auf den Halbleiterdetektor aufgebrachter Szintillator kann als separate biegbare oder gekrümmte Komponente ausgebildet sein, die auf den Halbleiterdetektor oder die faseroptische Platte oder die Gesamtvorderseite aufgebracht, insbesondere aufgeklebt, ist. Beispielsweise kann der Szintillator in Form einer Folie hergestellt bzw. bereitgestellt und auf die entsprechende Fläche aufgeklebt werden.

In jenem Fall, in dem der Halbleiterdetektor durch ein Substrat getragen wird, dessen dem Halbleiterdetektor zugewandte Seite und dessen von dem Halbleiterdetektor abgewandte Seite gekrümmt sind, kann auf die Nutzung der faseroptischen Platte auch verzichtet werden. Durch die Anordnung des Halbleiterdetektors am Substrat wird bei Nutzung von mehreren Halbleiterdetektoren deren relative Orientierung zueinander bereits festgelegt. Zudem ist der Halbleiterdetektor in diesem Fall vorzugsweise ebenfalls bereits gekrümmt, so dass bereits eine gleichmäßig gekrümmte Fläche bereitsteht, die unmittelbar mit dem Szintillator beschichtet werden kann bzw. auf die der Szintillator mit einem separaten Substrat aufgebracht werden kann.

Das den Halbleiterdetektor tragende Substrat kann eine Dicke zwischen 0,1 mm und 20 mm, bevorzugt zwischen 1 mm und 5 mm, aufweisen. Es kann aus Glas oder kristallinem Silizium oder aus einem Polymermaterial oder aus einer Keramik oder aus einem faserverstärkten Material bestehen oder wenigstens eines dieser Materialien umfassen. Beispielsweise kann es sich um ein Kompositmaterial handeln, das mehrere der genannten Materialien umfasst oder aus diesen besteht. Durch die genannten Parameter kann eine ausreichende Eigenstabilität des Röntgendetektors erreicht werden, so dass beispielsweise Störungen der Messung aufgrund von Vibrationen oder Verwindungen minimiert werden können. Sogleich wird die Baugröße des Röntgendetektors senkrecht zur Detektoroberfläche minimiert, so dass der Röntgendetektor beispielsweise mit geringem technischen Aufwand in die Röhre eines Magnetresonanztomographen integriert werden kann.

Ein an der Rückseite des Halbleiterdetektors angeordnetes Substrat kann auch vorteilhaft sein, wenn an faseroptischen Platten angeordnete Halbleiterdetektoren genutzt werden, um ein jeweiliges Detektormodul sowohl mechanisch als auch thermisch zu stabilisieren. Das Substrat kann zusätzlich zu dem fotoaktiven Halbleiterdetektor eine Auswerteelektronik tragen.

Der Röntgendetektor kann eine Detektorfläche von wenigstens 400 cm², insbesondere von wenigstens 800 cm² oder 1000 cm² aufweisen. Beispielsweise kann der Röntgendetektor eine Detektorfläche von 30 x 40 cm² aufweisen. Durch den erfindungsgemäßen Aufbau wird erreicht, dass der Röntgendetektor auch bei diesen relativ großen Abmessungen ausreichend stabil ist und eine gute Abbildungsqualität erreichen kann.

Das Substrat kann mehrere Leitungen ausbilden oder es können mehrere Leitungen durch das Substrat geführt sein, über die der Halbleiterdetektor kontaktiert ist. Beispielsweise kann ein Siliziumsubstrat genutzt werden, wobei die Kontaktierung des Halbleiterdetektors mit durch das Substrat geführten "through-silicon-vias" erfolgt. Das Substrat kann an jener Seite, an der der Halbleiterdetektor angeordnet wird, Kontaktflächen bzw. Kontaktpads aufweisen, mit denen Kontakte des Halbleiterdetektors verbunden werden können. Beispielsweise können Kontakte des Halbleiterdetektors unmittelbar mit diesen Kontaktflächen gebondet werden oder es kann ein Drahtbonding zwischen den Kontaktflächen und den Kontakten des Halbleiterdetektors erfolgen. Die Kontaktierung des Halbleiterdetektors kann gleichzeitig dazu dienen, die Position und/oder Form des Halbleiterdetektors vorzugeben. Beispielsweise kann der Halbleiterdetektor verformt werden, anschließend mit dem Substrat kontaktiert werden und die Verformung kann durch die Verbindung mit dem Substrat fixiert sein. Um eine dauerhafte Belastung der Kontakte zu vermeiden bzw. zu vermindern und den Halbleiterdetektor weiter zu stabilisieren, kann zudem nach der Kontaktierung der Zwischenraum zwischen Substrat und Halbleiterdetektor aufgefüllt werden, wobei insbesondere das gleiche Material genutzt wird, das auch das Substrat bildet.

Zwischen dem Halbleiterdetektor und der faseroptischen Platte und/oder zwischen der faseroptischen Platte und dem oder einem Szintillator und/oder zwischen dem Halbleiterdetektor und dem oder einem Szintillator kann eine zur Verminderung oder Reduzierung von Reflexionen an dem jeweiligen Materialübergang und insbesondere zur Verklebung dieser Komponenten dienende Kopplungsschicht angeordnet sein. Der Brechungsindex der Kontaktschicht kann derart gewählt werden, dass er zwischen den Brechungsindizes der angrenzenden Komponenten liegt, um Reflexionen an der Grenzschicht zu vermeiden bzw. zu vermindern.

Neben dem erfindungsgemäßen Röntgendetektor betrifft die Erfindung eine Röntgeneinrichtung, die eine Röntgenquelle und den erfindungsgemäßen Röntgendetektor umfasst. Bei der Röntgeneinrichtung kann es sich beispielsweise um eine Kombinationseinrichtung handeln, bei der der erfindungsgemäße Röntgendetektor in eine Röhre eines Magnetresonanztomographen oder einer Positronenemissionstomographieeinrichtung integriert ist. Die Krümmung der Detektoroberfläche bzw. des Szintillators, der Vorderseite und/oder der Rückseite der faserverstärkten Platte, des Halbleiterdetektors und/oder des Substrats kann so gewählt werden, dass die entsprechende Komponente bzw. Fläche die Form eines Zylindermantels bzw. eines Segments eines Zylindermantels aufweist, wobei der Mittelpunkt des Zylindermantels bzw. der entsprechenden Kreisfläche mit dem Isozentrum der weiteren Untersuchungseinrichtung zusammenfällt. Der Röntgendetektor kann beispielsweise zu Angiographiemessungen genutzt werden. Bei der Röntgeneinrichtung kann es sich jedoch auch um eine reine Röntgeneinrichtungen oder ein anderes Kombinationsgerät handeln.

Die Röntgeneinrichtung kann zusätzlich eine Magnetresonanzeinrichtung zur Erfassung von Magnetresonanzdaten umfassen, wobei der Röntgendetektor innerhalb eines von dem Hauptmagneten der Magnetresonanzeinrichtung umgriffenen, insbesondere zylinderförmigen, Hauptmagnetvolumen angeordnet ist. Der Röntgendetektor und/oder eine zugeordnete Ausleseelektronik können hierbei magnetresonanzkompatibel ausgelegt sein. Hierzu kann wenig, insbesondere weniger als ein Gramm, oder kein ferromagnetisches Material in dem Röntgendetektor verwendet werden. Ergänzend oder alternativ können die elektronischen Komponenten des Röntgendetektors durch eine Abschirmung abgeschirmt sein. Hierdurch kann eine Beeinflussung der Feldverteilung in der Magnetresonanzeinrichtung vermieden oder zumindest reduziert werden.

Die Magnetresonanzeinrichtung kann wenigstens eine Körperspule zum senden und/oder empfangen von Hochfrequenzsignalen, die innerhalb des Hauptmagnetvolumens angeordnet ist und einen insbesondere zylinderförmigen Untersuchungsbereich umgreift, und eine Gradientenspule zur Erzeugung von Gradientenfeldern, die zwischen der Körperspule und dem Hauptmagneten angeordnet ist, umfassen, wobei der Röntgendetektor zwischen der Gradientenspule und der Körperspule angeordnet ist. In diesem Fall kann durch den erfindungsgemäß aufgebauten Röntgendetektor eine geringe Bautiefe in Radialrichtung erreicht werden, wodurch vermieden wird, dass durch den zusätzlichen Röntgendetektor der Zugang zu einem Untersuchungsobjekt erschwert wird bzw. dass ein Hauptmagnet und eine Gradientenspule mit größerem Durchmesser genutzt werden müssen.

Eine Ausleseelektronik zum Auslesen des Halbleiterdetektors kann in Axialrichtung des Hauptmagnetvolumens oder des Untersuchungsvolumens versetzt neben dem Halbleiterdetektor angeordnet sein. Hierdurch kann ein Bauraumverbrauch in Radialrichtung gegenüber einer radial außenliegenden Anordnung der Ausleseelektronik verringert werden.

Eine Ausleseelektronik zum Auslesen des Halbleiterdetektors kann auf einem gekrümmten Schaltungsträger angeordnet sein. Dies kann dazu dienen, die Ausdehnung der zum Röntgen erforderlichen Komponenten senkrecht zur Detektoroberfläche weiter zu reduzieren. Die Elektronik kann beispielsweise auf der von dem Halbleiterdetektor abgewandten Substratseite angeordnet sein. Alternativ kann sie seitlich neben dem Halbleiterdetektor, insbesondere in Axialrichtung der entsprechenden Detektoranordnung, angeordnet sein. Die konkrete Anordnung der Elektronik kann in Abhängigkeit des vorhandenen Bauraums gewählt werden.

In wenigstens einer senkrecht auf der Vorderseite des Halbleiterdetektors stehenden Schnittebene können die in dieser Schnittebene liegenden Fasern der faseroptischen Platte in Radialrichtung bezüglich eines Fokuspunktes der Röntgenquelle oder bezüglich einer Geraden, die diesen Fokuspunkt umfasst, ausgerichtet sein. Die den Fokuspunkt umfassende Gerade kann in Axialrichtung des Hauptmagneten bzw. parallel zu der Detektoroberfläche bzw. der Vorderseite der faseroptischen Platte verlaufen. Durch eine derartige Faserführung können Verzerrungen der erfassten Röntgenbilder, zumindest in Umfangsrichtung, minimiert werden.

Die Röntgeneinrichtung kann eine Verarbeitungseinrichtung zur Verarbeitung von durch den Röntgendetektor erfassten Rohbilddaten umfassen, wobei die Verarbeitungseinrichtung dazu eingerichtet ist, die Rohbilddaten oder ein durch eine Vorverarbeitung aus den Rohbilddaten ermitteltes Zwischenbild in zwei, insbesondere orthogonale, Richtungen unterschiedlich stark zu dehnen oder zu stauchen oder in eine dieser Richtungen zu dehnen und in die andere zu stauchen, um eine unterschiedlich starke Krümmung des Röntgendetektors in die zwei Richtungen zu kompensieren. Hierdurch können beispielsweise unterschiedliche Krümmungsradien bzw. Faserausrichtungen in Axialrichtung und in Umfangsrichtung des Röntgendetektors zumindest weitgehend kompensiert werden.

Der Halbleiterdetektor kann derart an der faseroptischen Platte angeordnet sein, dass erste Fasern der faseroptischen Platte von einem Bildgebungsbereich des Halbleiterdetektors zu der von dem Halbleiterdetektor abgewandten Vorderseite der faseroptischen Platte geführt sind und dass zweite Fasern der faseroptischen Platte von einem Referenzbereich des Halbleiterdetektors zu der oder einer weiteren Seitenfläche der faseroptischen Platte geführt sind, wobei die Röntgeneinrichtung die oder eine Verarbeitungseinrichtung umfasst, die dazu eingerichtet ist, Ergebnisbilddaten aus durch den Röntgendetektor erfassten Rohbilddaten zu ermitteln, wobei die Bilddaten wenigstens eines Bildpunktes der Ergebnisbilddaten sowohl von Rohbilddaten abhängen, die im Bildgebungsbereich des Halbleiterdetektors erfasst werden, als auch von Rohbilddaten, die in dem Referenzbereich des Halbleiterdetektors erfasst werden. Da der Szintillator typischerweise nur an der Vorderseite der faseroptischen Platte vorgesehen wird, sollte bei einem idealen Verhalten der faseroptischen Platte und des Halbleiterdetektors in dem Referenzbereich keine Intensität erfasst werden. Die im Referenzbereich gemessene Intensität ist daher eine Messgröße, die Abweichungen des realen Röntgendetektors von diesem Idealen Verhalten, insbesondere aufgrund eines geringfügigen Übersprechens zwischen den Fasern bzw. von Signalen im Halbleiterdetektor. Da somit durch die Vergrößerung des Halbleiterdetektors in den Referenzbereich hinein entsprechende Abweichungen quantifiziert werden können, können die Messgrößen zur Korrektur der Bilddaten genutzt werden. Andererseits kann, insbesondere zur Kostenersparnis, ein kleinerer Halbleiterdetektor genutzt werden, der ausschließlich im Bildbereich Messdaten erfasst, ohne dass Bildinformationen verloren gehen.

Zudem betrifft die Erfindung ein Verfahren zur Herstellung eines Röntgendetektors mit einer konvexen oder im Wesentlichen hohlzylindersegmentförmigen Detektoroberfläche, indem entweder mehrere Halbleiterdetektoren bereitgestellt werden, deren der Detektoroberfläche zugewandte Vorderseite jeweils an einer dem jeweiligen Halbleiterdetektor zugeordneten faseroptische Platte angebracht wird, wobei faseroptische Platten verwendet werden, die eine gekrümmte von dem Halbleiterdetektor abgewandte Vorderseite aufweisen und/oder die jeweils wenigstens eine Seitenfläche aufweisen, die nach dem Anbringen der faseroptischen Platte an der Vorderseite des zugeordneten Halbleiterdetektors einen spitzen Winkel mit der Vorderseite einschließt, wobei diese Seitenfläche mit einer Seitenfläche einer weiteren der faseroptischen Platten verbunden wird, oder indem ein bereitgestellter Halbleiterdetektor gekrümmt und an einer gekrümmten Halteeinrichtung angebracht wird, wonach der Halbleiterdetektor mit Kontakten eines Substrats, dessen dem Halbleiterdetektor zugewandte Seite und von dem Halbleiterdetektor abgewandte Seite gekrümmt sind, elektrisch kontaktiert wird, wonach der Halbleiterdetektor von der Halteeinrichtung abgelöst wird.

In der ersten Variante wird die Anordnung der Halbleiterdetektoren bzw. die Form der Detektoroberfläche durch die Formgebung der faseroptischen Platten vorgegeben. Diese können beispielsweise durch eine spanabhebende Bearbeitung geformt werden oder unmittelbar in der gewünschten Form hergestellt werden. Komponenten können im Rahmen der Herstellung z.B. durch Verkleben verbunden werden. Auf die Vorderseite der faseroptischen Platten kann, wie bereits erläutert, ein Szintillator aufgebracht werden, z.B. als eine Folie aufgeklebt werden oder Ähnliches.

Wie bereits vorangehend erläutert, wird im Rahmen der zweiten Variante die Form des Halbleiterdetektors durch den Kontakt mit dem Substrat vorgegeben. Hierbei kann alleine die elektronische Kontaktierung den Halbleiterdetektor in Form halten, es ist jedoch auch möglich, vor oder nach dem Ablösen des Halbleiterdetektors von der Halteeinrichtung den Zwischenraum zwischen Substrat und Halbleiterdetektor mit Material, insbesondere mit dem Material des Substrats, aufzufüllen. Hierdurch kann eine Belastung der elektronischen Kontakte verringert werden und die Form und Position des Halbleiterdetektors kann weiter stabilisiert werden.

Ein Szintillator kann bereits auf den bereitgestellten Halbleiterdetektor aufgebracht sein. Besonders bevorzugt wird der Szintillator jedoch erst nach Entfernen der Halteeinrichtung aufgebracht. Der Szintillator kann unmittelbar auf den Halbleiterdetektor aufgebracht werden. Alternativ ist es jedoch möglich, zunächst eine faseroptische Platte an den Halbleiterdetektor anzuordnen und den Szintillator auf die von dem Halbleiterdetektor abgewandte Oberfläche dieser faseroptischen Platte aufzubringen. Wie vorangehend erläutert kann der Szintillator unmittelbar oder unter Verwendung einer Bindeschicht auf den Halbleiterdetektor bzw. die faseroptische Platte aufgedampft werden. Alternativ kann er auch auf ein durchsichtiges Substrat aufgebracht und mit dem Halbleiterdetektor bzw. der faseroptischen Platte verklebt werden.

Die Halterung des Halbleiterdetektors an der Halteeinrichtung kann durch Verkleben erfolgen. Insbesondere kann eine thermisch lösbare Verklebung, beispielsweise ein thermisch lösbares Klebeband, verwendet werden. Das Verfahren kann insbesondere zur Herstellung eines erfindungsgemäßen Röntgendetektors genutzt werden.

Weitere Vorteile und Einzelheiten der Erfindung zeigen die folgenden Ausführungsbeispiele sowie die zugehörigen Zeichnungen. Hierbei zeigen schematisch:
- Fig. 1: ein Ausführungsbeispiel einer erfindungsgemä-ßen Röntgeneinrichtung,
- Fig. 2: ein Ausführungsbeispiel eines erfindungsgemäßen Röntgendetektors,
- Fig. 3 - 5: Detailansichten der faseroptischen Platte und des Halbleiterdetektors in verschiedenen Ausführungsvarianten des in Fig. 2 gezeigten Röntgendetektors,
- Fig. 6 - 10: weitere Ausführungsbeispiele des erfindungsgemäßen Röntgendetektors,
- Fig. 11 - 14: verschiedene Schrittes eines Ausführungsbeispiels des erfindungsgemäßen Verfahrens zur Herstellung eines Röntgendetektors, und
- Fig. 15 und 16: Detailansichten eines weiteren Ausführungsbeispiels eines erfindungsgemäßen Röntgendetektors.

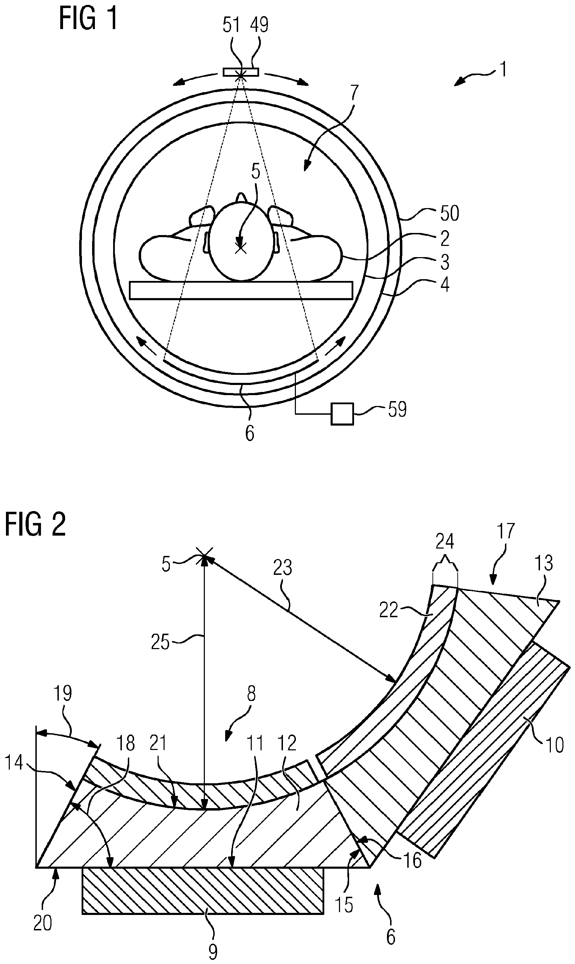

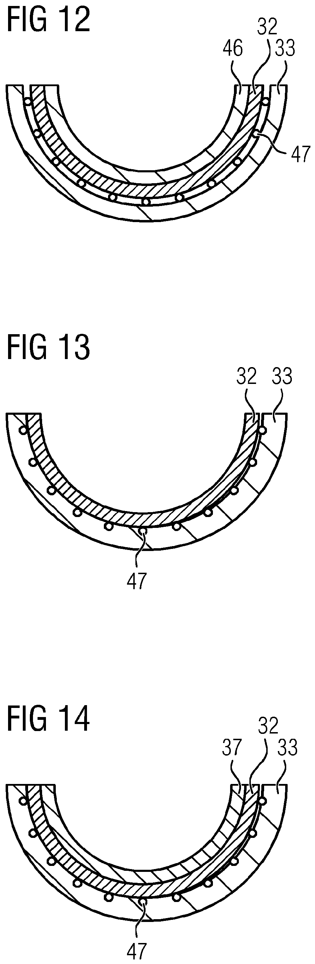

Fig. 1 zeigt eine Röntgeneinrichtung 1 zur Erfassung von Messdaten bezüglich eines Untersuchungsobjekts 2, insbesondere eines Patienten. Die Röntgeneinrichtung 1 ist in dem Ausführungsbeispiel eine kombinierte Bildgebungseinrichtung, die einerseits Röntgendaten, beispielsweise im Rahmen einer Angiographiemessung, und andererseits Magnetresonanzdaten erfassen kann. Zur Erfassung der Magnetresonanzdaten sind, neben weiteren nicht gezeigten Komponenten, eine Körperspule 3 und eine Gradientenspule 4 im Wesentlichen zylinderförmig um ein Isozentrum 5 herum angeordnet. Um eine einfache Registrierung der Messdaten zu ermöglichen, werden auch die Röntgendaten durch einen Röntgendetektor 6 erfasst, der sich im Wesentlichen zylindersegmentförmig um das Isozentrum 5 herum erstreckt. Die zugehörige Röntgenquelle 49 kann, wie durch die entsprechenden Pfeile dargestellt ist, gemeinsam mit dem Röntgendetektor 6 um das Isozentrum 5 verschwenkt werden. In dem gezeigten Ausführungsbeispiel ist der Röntgendetektor 6 zwischen der Körperspule 3 und der Gradientenspule 4 angeordnet, die beide innerhalb des Hauptmagneten 50 angeordnet sind. Hierbei ist es wesentlich, dass der Röntgendetektor 6 eine möglichst geringe Ausdehnung in Radialrichtung aufweist, da ein relativ dicker Röntgendetektor 6 dazu führen würde, dass der Durchmesser der Röhre 7 erweitert werden muss, wodurch der technische Aufwand und die Kosten zur Implementierung einer Magnetresonanzmessung erheblich steigen. Möglichkeiten, einen Röntgendetektor mit einer zylinderförmigen bzw. zylindersegmentförmigen Detektoroberfläche zu implementieren, werden detailliert mit Bezug auf die Figuren 2 bis 14 beschrieben werden.

Während das Ausführungsbeispiel in Fig. 1 eine Röntgeneinrichtung zeigt, die in einen Magnetresonanztomographen integriert ist, ist es selbstverständlich möglich, den Röntgendetektor 6 auch für sich genommen als reine Röntgeneinrichtung zu nutzen und/oder ihn mit anderen Bildgebungsmodalitäten, beispielsweise mit einer Positronenemissionstomographie, zu kombinieren. Hierbei kann eine zylindersegmentförmige Detektoroberfläche genutzt werden, wie sie in Fig. 1 dargestellt ist, es ist jedoch auch möglich von der Zylinderflächen- bzw. Kreisform abzuweichen, um anderweitig gekrümmte Röntgendetektoren 6 bereitzustellen.

Fig. 2 zeigt eine Möglichkeit, wie eine konvexe bzw. im Wesentlichen hohlzylindrische Detektoroberfläche 8 des Röntgendetektors 6 mit relativ geringem technischen Aufwand implementiert werden kann. Hierbei werden mehrere Halbleiterdetektoren 9, 10 beispielsweise CMOS-Detektoren, genutzt, an deren der Detektoroberfläche 8 zugewandten Vorderseiten 11 jeweils eine faseroptische Platte 12, 13 angeordnet ist. Die faseroptischen Platten 12, 13 weisen jeweils zwei Seitenflächen 14, 15, 16, 17 auf, die einen spitzen Winkel 18 mit der Vorderseite 11 des zugeordneten Halbleiterdetektors 9, 10 einschließen. Über die Seitenflächen 15, 16 sind die faseroptischen Platten 12, 13 miteinander verbunden. Hierdurch wird ein definierter Winkel zwischen den Vorderseiten 11 der Halbleiterdetektoren 9, 10 vorgegeben, der dem Doppelten des Winkels 19 entspricht, also dem Doppelten jenes Winkels, um den der Winkel 18 zwischen den Seitenflächen 14, 15, 16, 17 und der Vorderseite 11 des jeweils zugeordneten Halbleiterdetektors 9, 10 von der Senkrechten abweicht.

Die Detektoroberfläche 8 erfasst im gezeigten Ausführungsbeispiel einen Raumwinkel von ca. 60°. Sollen größere Raumwinkel erfasst werden, können an den Seitenflächen 14 bzw. 17 weitere faseroptischen Platten mit zugeordneten Halbleiterdetektoren angeordnet werden. Durch die Nutzung der faseroptischen Platten 12, 13 mit den spitzwinkligen Seitenflächen 14, 15, 16, 17 wird es somit mit geringem technischen Aufwand ermöglicht, mit hoher Genauigkeit eine relative Orientierung von mehreren Halbleiterdetektoren 9, 10 zueinander festzulegen.

Die faseroptischen Platten 12, 13 weisen jeweils eine Ebene, dem jeweiligen Halbleiterdetektor 9, 10 zugewandte Rückseite 20 auf. Dies ermöglicht die Nutzung von flachen Halbleiterdetektoren 9, 10, die mit geringem Aufwand herstellbar sind.

Auf die von dem jeweiligen Halbleiterdetektor 9, 10 abgewandte Vorderseite 21 der faseroptischen Platten 12, 13 ist jeweils eine Szintillatorschicht 22 aufgebracht, die bei einem Auftreffen von Röntgenstrahlung Licht abgibt, das in die faseroptische Platte 12, 13 eingespeist wird. Um zu ermöglichen, dass die Detektoroberfläche 8 einen im Wesentlichen konstanten Radius 23 aufweist und zugleich ein Szintillator 22 mit im Wesentlichen konstanter Dicke 24 nutzbar ist, ist die von den Halbleiterdetektoren 9, 10 abgewandte Vorderseite 21 der faseroptischen Platten 12, 13 hohlzylindersegmentförmig ausgebildet und weist einen festen Krümmungsradius 25 auf. Hierdurch können Variationen der Detektorempfindlichkeit aufgrund variierender Szintillatordicken vermieden werden.

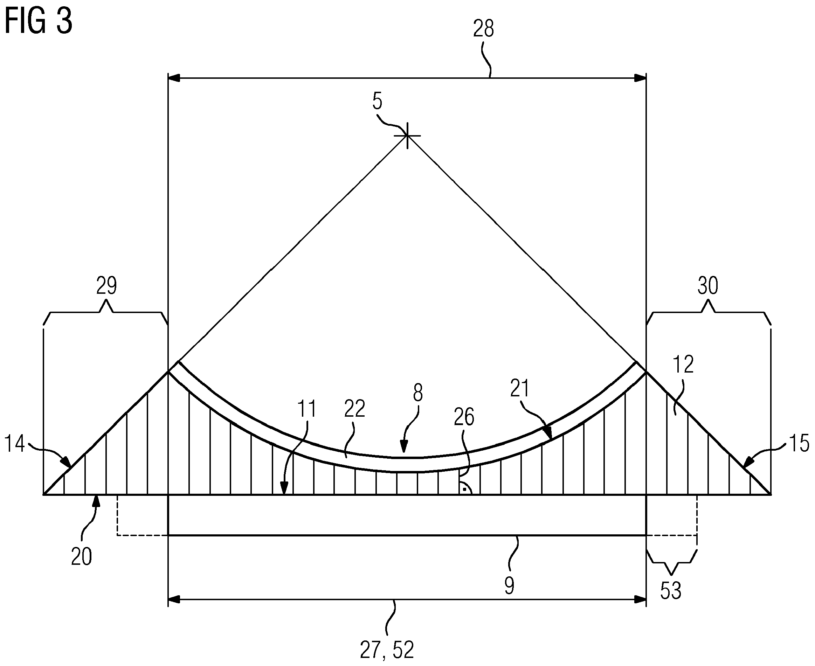

Fig. 3 zeigt schematisch Details des Aufbaus der faseroptischen Platte 12. Die Seitenflächen 14, 15 erstrecken sich radial bezüglich des Isozentrums 5. Die faseroptische Platte ist durch eine Vielzahl paralleler Fasern 26 gebildet, die sich im Wesentlichen senkrecht zu der Vorderseite 11 des Halbleiterdetektors 9 erstrecken. Die Fasern können beispielsweise Glasfasern sein oder aus einem Polymer bestehen. Aus Übersichtlichkeitsgründen sind relativ wenige, stark beabstandete Fasern 26 dargestellt. Bei einer realen Umsetzung werden jedoch typischerweise Fasern mit einer Faserdicke im Bereich von einigen µm bis einigen 100 µm genutzt, womit eine hochauflösende Datenerfassung ermöglicht wird.

Die Rückseite 20 der faseroptischen Platte 12 ragt über den Halbleiterdetektor 9 hinaus. Die Breite 27 des Halbleiterdetektors 9 entspricht hierbei im Wesentlichen der Breite 28 der Vorderseite 21 der faseroptischen Platte 12. Da ausschließlich diese Vorderseite 21 durch den Szintillator 22 bedeckt ist, womit in die seitlichen Bereiche 29, 30 im Wesentlichen kein durch den Szintillator 22 emittiertes Licht eingekoppelt wird, wird aufgrund der senkrecht auf der Vorderseite 11 des Halbleiterdetektors 9 stehenden Fasern 26 an der Rückseite 20 der faseroptischen Platte 12 in den Bereichen 29, 30 im Wesentlichen kein durch Röntgenstrahlung ausgelöstes Licht emittiert, so dass eine Erstreckung des Halbleiterdetektors 9 in diese Bereiche unnötig ist.

Die Ausführungsform gemäß Fig. 3 ist vorteilhaft, da eine faseroptische Platte 12 mit im Wesentlichen parallelen Fasern 26 relativ einfach herstellbar ist. Die gezeigte Konfiguration führt jedoch dazu, dass entlang der Detektoroberfläche 8 bzw. der Vorderseite 21 der faseroptischen Platte 12 die Ortsauflösung des Detektors variiert. Dies kann zu Verzerrungen der resultierenden Bilddaten führen, wobei Aufgrund der Messgeometrie verschieden starke Verzerrungen in Umfangsrichtung und in Axialrichtung der Messröhre auftreten. Eine Entzerrung, die dies Berücksichtigt, kann durch die in Fig. 1 dargestellte Verarbeitungseinrichtung 59 erfolgen.

In realen faseroptischen Platten erfolgt ein geringfügiges Übersprechen zwischen den Fasern, das zu einer Kontrastreduzierung führen kann. Auch dieses Übersprechen kann durch die Verarbeitungseinrichtung 59 zumindest teilweise kompensiert werden. Hierzu ist es jedoch Vorteilhaft, wenn die Breite 27 des Halbleiterdetektors 9 in Fig. 3 etwas erhöht wird. In einem Referenzbereich 53 kann somit erfasst werden, wieviel Licht durch Fasern 26 übertragen wird, die nicht an der Vorderseite 21 der faseroptischen Platte sondern an deren Seitenflächen 14, 15 enden. Dies ist ein Maß für das Übersprechen zwischen den Fasern, das im Rahmen der Datenkorrektur ausgewertet werden kann, beispielsweise um eine Entfaltung bezüglich eines aus den Intensitäten im Referenzbereich 53 ermittelten Faltungs-Kernel zu berechnen. In dem Bildbereich 52 werden hingegen die Bildinformationen erfasst, deren Kontrast mit Hilfe der in dem Referenzbereich 53 ermittelten Daten verbessert werden kann.

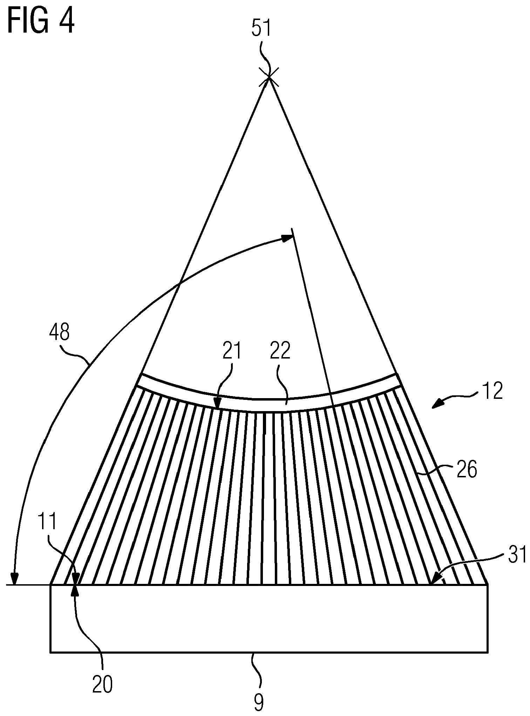

Um die beschriebenen Verzerrungen zu minimieren kann es vorteilhaft sein, wenn der Winkel 48 zwischen den Fasern 26 und der Vorderseite 11 des Halbleiterdetektors 9 in wenigstens einer Richtung monoton mit der Position des halbleiterdetektorseitigen Endes 31 der Fasern 26 variiert. Ein Beispiel hierfür ist in Fig. 4 dargestellt. Im gezeigten Beispiel sind die Orientierungen der Fasern 26 so gewählt, dass die einzelnen Fasern 26 senkrecht auf der Vorderseite 21 der faseroptischen Platte 12 stehen. Hierdurch kann eine im Wesentlichen ortsunabhängige Auflösung des Röntgendetektors erreicht werden, wenn ein Fokuspunkt 25 der Röntgenquelle 49 den Mittelpunkt des durch die Detektorfläche 8 gebildeten Kreises bildet. Hierbei wird vorzugsweise ein Halbleiterdetektor 9 genutzt, der die gesamte Rückseite 20 der faseroptischen Platte 12 bedeckt.

Bei einer Nutzung in einer Magnetresonanzeinrichtung ist der Mittelpunkt des Kreises der Detektorfläche jedoch typischerweise das Isozentrum 5, wobei der Fokuspunkt 51 weiter von der Detektorfläche entfernt ist, als das Isozentrum 5. Zwar kann auch in diesem Fall eine senkrechte Ausrichtung der Fasern bezüglich der Vorderseite 21 der faseroptischen Platte eine Bildverzerrung gegenüber einer senkrechten Ausrichtung bezüglich der Vorderseite des Halbleiterdetektors reduzieren. Es kann jedoch vorteilhaft sein, die Fasern so anzuordnen, dass sie radial bezüglich des Fokuspunkts 25 bzw. bezüglich einer insbesondere senkrecht auf der Bildebene der Fig. 4 stehenden Geraden durch den Fokuspunkt 25 liegen.

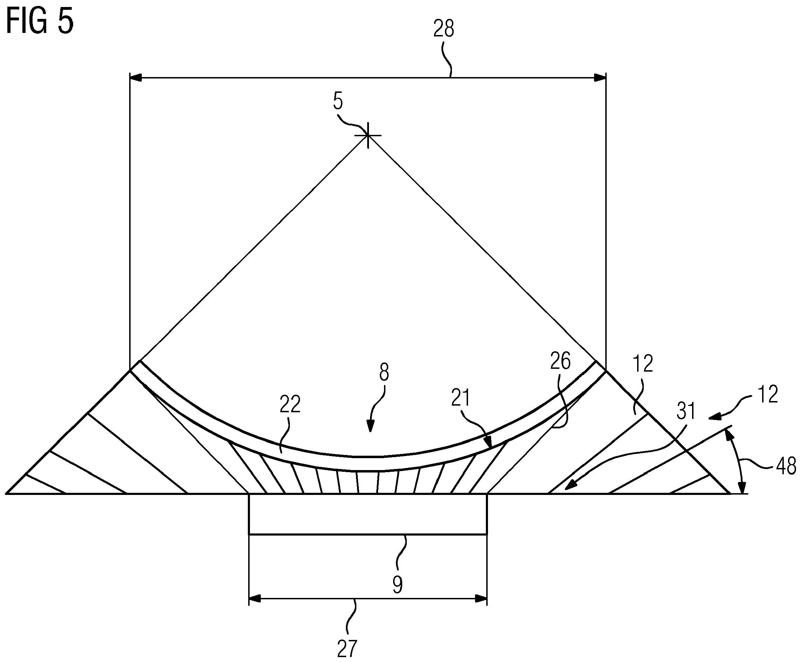

In einigen Fällen kann es gewünscht sein, relativ kleine Halbleiterdetektoren 9 zu nutzen, beispielsweise um die Kosten des Röntgendetektors zu reduzieren. Ein Beispiel hierfür ist in Fig. 5 dargestellt. In dieser wird ein Halbleiterdetektor 9 genutzt, dessen Breite 27 kleiner ist als die Breite 28 der Vorderseite 21 der faseroptischen Platte 12. Um dennoch auf der gesamten Detektoroberfläche 8 einfallende Röntgenstrahlung detektieren zu können, wird die Ausrichtung der Fasern 26 in der faseroptischen Platte wiederum monoton mit der Position des halbleiterdetektorseitigen Endes 31 der jeweiligen Faser variiert. Diese Variation erfolgt jedoch umgekehrt als in dem in Fig. 4 dargestellten Ausführungsbeispiel. Somit resultiert zwar eine starke Variation der lokalen Auflösung entlang der Detektoroberfläche 8, es wird jedoch trotz relativ kleiner Fläche des Halbleiterdetektors 9 die gesamte Detektoroberfläche 8 abgebildet.

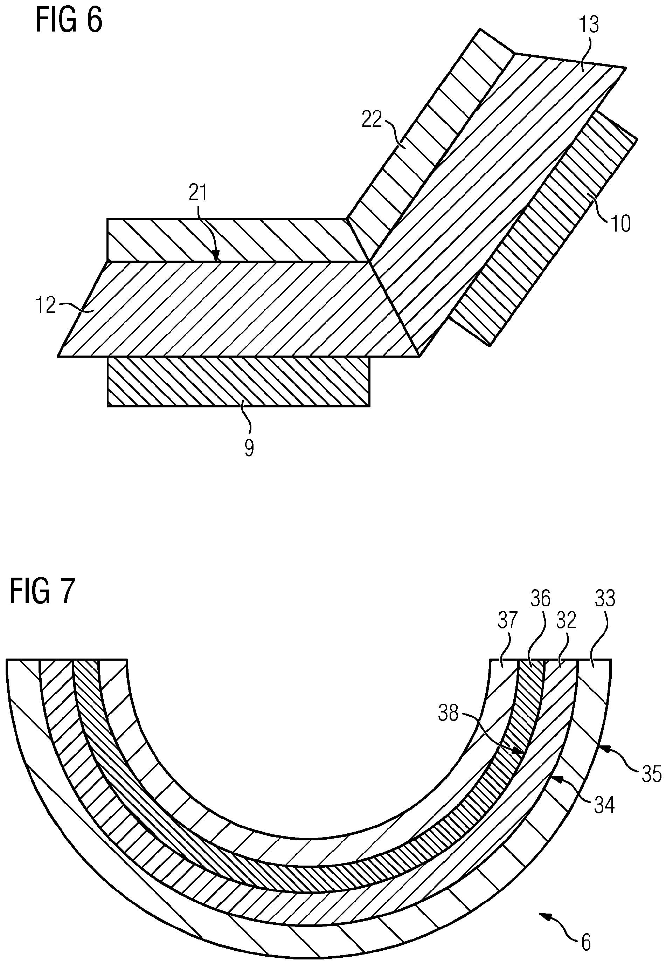

Die mit Bezug auf die Figuren 2 bis 5 erläuterten Ausführungsbeispiele nutzen jeweils eine gekrümmte Vorderseite 21 der faseroptischen Platten 12, 13. Dies ist vorteilhaft, da hierdurch erreicht werden kann, dass Röntgenstrahlung im Wesentlichen jeweils die gleiche Szintillatordicke durchquert und eine Lichtemission des Szintillators durch einen einzelnen Röntgenstrahl primär durch einzelne oder eine kleine Gruppe von Fasern erfasst und somit in einen kleinen Bereich des jeweiligen Röntgendetektors 9, 10 geführt wird. Zugleich erhöht jedoch die Nutzung einer gekrümmten Vorderseite 21 der faseroptischen Platten 12, 13 den Herstellungsaufwand für den Röntgendetektor. In einigen Fällen kann es daher gewünscht sein, eine flache Vorderseite 21 der faseroptischen Platten 12, 13 zu nutzen, wie dies in Fig. 6 beispielhaft dargestellt ist. Die hieraus resultierenden Abbildungsfehler können beispielsweise im Rahmen einer nachträglichen Bildverarbeitung weitgehend kompensiert werden.

In den bisherigen Ausführungsbeispielen wurde jeweils davon ausgegangen, dass separate Szintillatoren 22 für die einzelnen faseroptischen Platten 12, 13 vorgesehen sind. Dies ermöglicht eine besonders einfache Herstellung des Röntgendetektors, da zunächst separate Module hergestellt werden können, die eine jeweilige faseroptische Platte 12, 13 mit daran angeordnetem Halbleiterdetektor 9, 10 und Szintillator 22 umfassen. Diese müssen anschließend nur noch zusammengefügt, beispielsweise miteinander verklebt, werden. Zur Herstellung einer besonders glatten Oberfläche und zur robusten Versiegelung dieser, beispielsweise um zu vermeiden, dass der Szintillator 22 Wasser zieht, kann es jedoch vorteilhaft sein, den Szintillator 22 erst nach Zusammenfügen mehrerer der faseroptischen Platten 12, 13 bzw. alle faseroptischen Platten 12, 13 aufzubringen. Der Szintillator kann in diesem Fall beispielsweise auf eine durch die Vorderseiten 21 der faseroptischen Platten 12, 13 gebildete Gesamtoberfläche unmittelbar oder mit Hilfe einer Binderschicht aufgedampft werden. Alternativ ist es beispielsweise möglich, den Szintillator zunächst auf ein separates durchsichtiges Substrat aufzubringen und dieses auf die Oberflächen der einzelnen faseroptischen Platten 12, 13 bzw. auf die Gesamtoberfläche aufzukleben.

Die Ausführungsbeispiele gemäß der Figuren 2 bis 6 genutzten jeweils ebene Halbleiterdetektoren 9, 10, um konvexe bzw. im Wesentlichen hohlzylindersegmentförmige Detektoroberflächen 8 bereitzustellen. Die folgenden Ausführungsbeispiele nutzen hingegen unmittelbar gekrümmte Halbleiterdetektoren. Es ist hierbei aus dem Bereich kleinflächiger Detektoren bekannt, dass CMOS-Detektoren, wenn sie auf eine ausreichend geringe Dicke von beispielsweise weniger als 500 µm geschliffen werden, biegbar sind und beispielsweise Biegeradien von 45 cm oder 35 cm erreicht werden können. Um derartig gekrümmte Detektoren auch für großflächige, beispielsweise zylindersegmentförmige oder halbkreisförmige, Detektoren nutzen zu können, wird, wie in Fig. 7 gezeigt ist, der gekrümmte Halbleiterdetektor 32 an einem Substrat 33, das beispielsweise aus Glas, Silizium oder Metall besteht, befestigt. Sowohl die dem Halbleiterdetektor 32 zugewandte Seite 34 als auch die von dem Halbleiterdetektor 32 abgewandte Seite 35 des Substrats sind hierbei gekrümmt, vorzugsweise mit einem Krümmungsradius, der dem Abstand von einem Isozentrum der Röntgeneinrichtung entspricht. Hierdurch wird insgesamt ein sehr platzsparender Aufbau in Radialrichtung des Röntgendetektors erreicht. An der von dem Substrat 33 abgewandten Vorderseite 38 des Halbleiterdetektors 32 sind eine gekrümmte, insbesondere zylindersegmentförmige, faseroptische Platte 36 und ein darauf angeordneter Szintillator 37 angeordnet. Die Fasern der faseroptischen Platte 36 stehen insbesondere senkrecht auf der Vorderseite 38 des Halbleiterdetektors 32 und verlaufen somit im Wesentlichen radial bezüglich der zylinderartigen Anordnung der Komponenten.

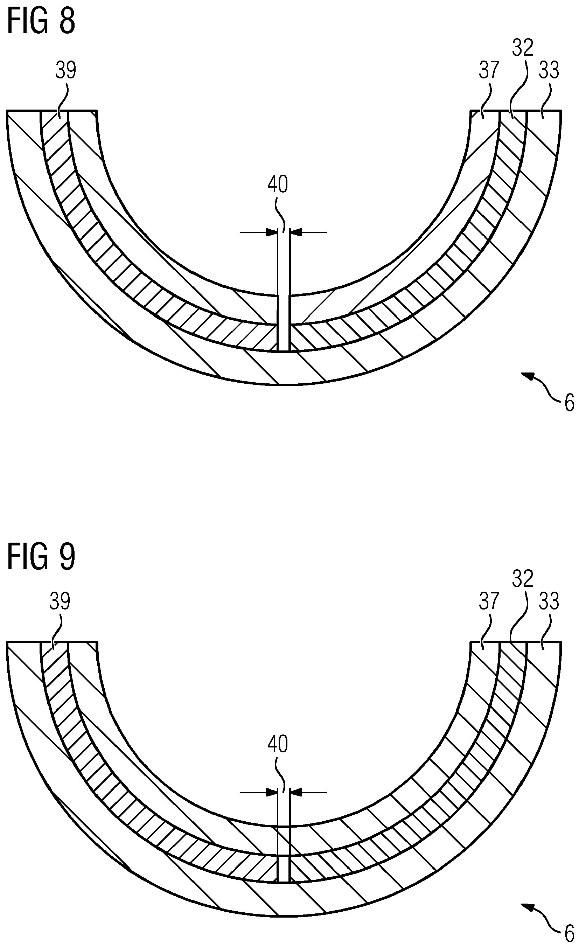

Fig. 8 zeigt eine geringfügige Abwandlung des in Fig. 7 gezeigten Röntgendetektors 6. Statt eines einzigen Halbleiterdetektors 32 werden in diesem Ausführungsbeispiel mehrere Halbleiterdetektoren 32, 39 genutzt. Um eine Nichtabbildung bestimmter Bereiche zu vermeiden, ist ein Abstand 40 zwischen den Halbleiterdetektoren vorzugsweise kleiner gewählt als die Ausdehnung eines jeweiligen Bildpunktes der Halbleiterdetektoren 32, 39.

Das Ausführungsbeispiel gemäß Fig. 8 unterscheidet sich zudem von dem Ausführungsbeispiel gemäß Fig. 7 dadurch, dass ein jeweiliger Szintillator 37 unmittelbar auf dem jeweiligen Halbleiterdetektor 32, 39 angeordnet ist. Dieser kann sich auch, wie in Fig. 9 gezeigt ist, über den Abstand 40 zwischen den Halbleiterdetektoren 32, 39 hinweg erstrecken, beispielsweise wenn der Szintillator 37 nach der Anordnung der Halbleiterdetektoren 32, 39 auf deren Oberfläche aufgedampft oder aufgeklebt wurde.

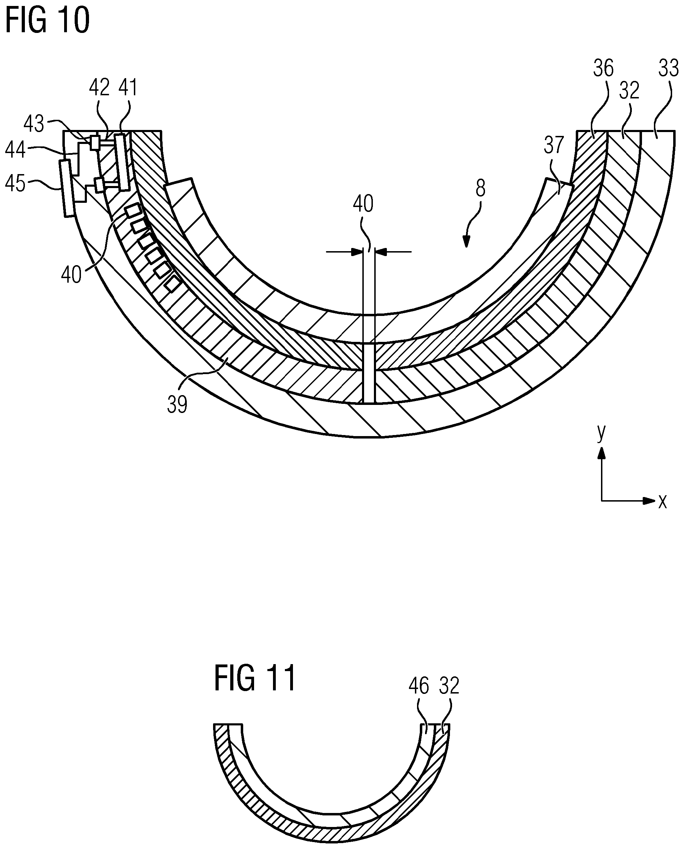

Wie in Fig. 10 schematisch dargestellt ist, kann das Substrat 33 nicht nur zur mechanischen Halterung und Formvorgabe für die Halbleiterdetektoren 32, 39 dienen, sondern auch zur elektrischen Kontaktierung. Die nur schematisch dargestellten Bildpunktdetektoren 40 werden durch eine Ausleseelektronik 41 ausgelesen, die in dem gezeigten Ausführungsbeispiel in Umfangsrichtung gegenüber der Detektoroberfläche 8, die durch die Oberfläche des Szintillators 37 gebildet wird, versetzt ist. Alternativ könnte die Ausleseelektronik 41 auch in Axialrichtung, also senkrecht zur Bildebene, gegenüber den Bildpunktdetektoren 40 versetzt sein oder an der Außenseite des Substrats 33 angeordnet sein. Die Ausleseelektronik 41 ist der Form des Halbleiterdetektors 39 angepasst, indem sie auf einem gekrümmten Schaltungsträger angeordnet ist.

Signalleitungen 42 dieser Ausleseelektronik 41 sind zur Rückseite des Halbleiterdetektors 39 geführt und dort, beispielsweise durch Bonden, mit Kontakten 43 des Substrats 33 leitend verbunden. Diese leitende Verbindung kann zusätzlich zur mechanischen Fixierung des Halbleiterdetektors 39 am Substrat 33 dienen, wobei diese Fixierung, wie im Folgenden noch genauer erläutert werden wird, durch eine Verfüllung verbleibender Zwischenräume zwischen Substrat 33 und Halbleiterdetektor 39 verbessert werden kann.

Durch das Substrat 33 sind mehrere Leitungen 44 geführt, die die Kontakte 43 mit einem Steckverbinder 45 verbinden. In alternativen Ausgestaltungen könnten beispielsweise diese Leitungen 44 auch dazu dienen, eine an der Außenseite des Substrats 33 angeordnete Ausleseelektronik mit den einzelnen Bildpunktdetektoren 40 zu verbinden. Die Leitungen können beispielsweise metallische Leitungen sein, die in einem Glassubstrat eingegossen sind, es können Durchkontaktierungen eines Silikonsubstrats verwendet werden oder Ähnliches.

In den Figuren 11 bis 14 sind verschiedene Schritte eines Herstellungsverfahrens visualisiert, das zur Herstellung eines Röntgendetektors mit gekrümmtem Halbleiterdetektor genutzt werden kann. Hierbei wird zunächst ein Halbleiterdetektor 32, der insbesondere durch einen CMOS-Prozess hergestellt wurde, bereitgestellt. Dieser wird ausreichend dünn ausgebildet bzw. wird ausreichend dünn geschliffen, dass er bis zu einem gewünschten Biegeradius von beispielsweise 35 cm oder 45 cm biegbar ist. Der Halbleiterdetektor wird anschließend, wie in Fig. 11 dargestellt ist, an einer gekrümmten Halteeinrichtung 46 mit dem gewünschten Biegeradius fixiert, beispielsweise durch thermolösbares Klebeband angeklebt.

Der so vorgeformte Halbleiterdetektor 32 wird anschließend benachbart zu dem Substrat 33 angeordnet und durch mehrere Kontakte 47, die insbesondere zumindest teilweise zur elektrischen Kontaktierung des Halbleiterdetektors 32 dienen können, an diesem fixiert. Die Kontakte 47 können beispielsweise dadurch implementiert werden, dass halbleiterdetektorseitig und substratseitig jeweils metallische Kontaktflächen vorgesehen sind, die durch ein Bonden verbunden werden.

Durch die Kontakte 47 ist die Form des Halbleiterdetektors 32 fixiert. Zudem kann diese Verbindung zur elektrischen Kontaktierung dienen. Daher wäre es prinzipiell bereits jetzt möglich, die Halteeinrichtung 46, beispielsweise durch thermisches Lösen der Verklebung, von dem Halbleiterdetektor 32 abzulösen und zu entfernen. Bevorzugt wird jedoch zunächst der Zwischenraum zwischen dem Substrat 33 und dem Halbleiterdetektor 32 verfüllt, vorzugsweise durch das gleiche Material, durch das auch das Substrat 33 gebildet ist. Es können jedoch auch andere Materialien genutzt werden. Beispielsweise kann eine Verfüllung durch Kunststoff oder Ähnliches erfolgen. Hierdurch wird die Form und Lage des Halbleiterdetektors 32 weiter stabilisiert, vorzugsweise bevor die Halteeinrichtung 46 entfernt wird.

Auf die nun freiliegende Oberfläche des Halbleiterdetektors 32 kann nun unmittelbar der Szintillator 37 aufgebracht werden, beispielsweise durch ein Aufdampfen. Alternativ wäre es möglich, zunächst eine faseroptische Platte auf dem Halbleiterdetektor 32 anzuordnen und erst auf diese den Szintillator 37 aufzubringen.

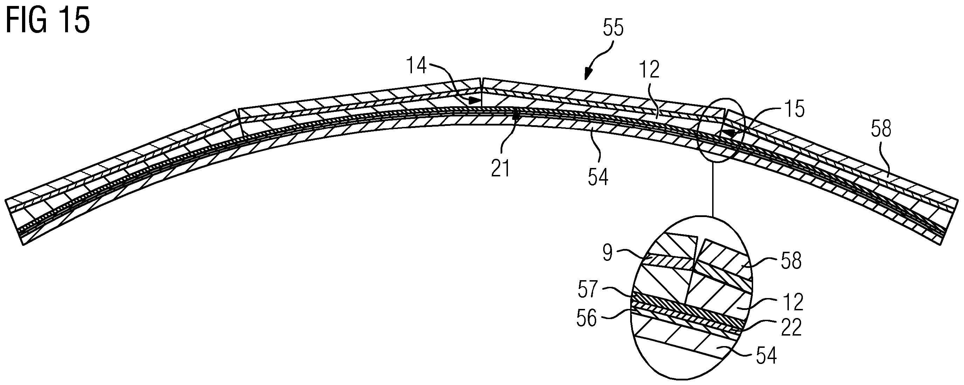

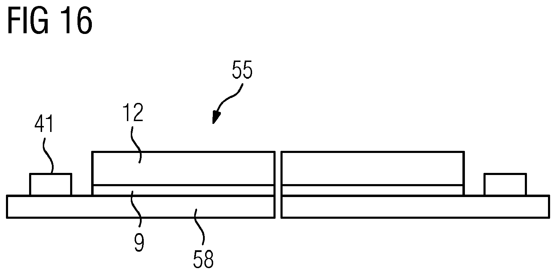

Fig. 15 und 16 zeigen Detailansichten eines weiteren Röntgendetektors, der mehrere an einem Substrat 54 angeordnete Detektormodule 55 umfasst. Dieser wird aufgebaut, indem zunächst ein gekrümmtes und für Röntgenstrahlung durchlässiges Substrat 54 bereitgestellt wird. Auf dieses wird mit Hilfe einer Klebeschicht 56 ein Szintillator 22 aufgebracht. Dieser kann z.B. als gekrümmte Komponente oder als Folie aufgeklebt werden. Auf die von dem Substrat abgewandte Seite des Szintillators 22 wird eine Kopplungsschicht 57 aufgebracht, insbesondere ein Kleber. Der Brechungsindex der Kopplungsschicht 57 kann so gewählt werden, dass im zusammengesetzten Röntgendetektor Reflexionen an der Grenzfläche zwischen der faseroptischen Platte 12 und dem Szintillator 22 minimiert werden. Die einzelnen Detektormodule 55 werden jeweils mit der Vorderseite ihrer faseroptischen Platte 12 auf diese Kopplungsschicht 57 aufgesetzt und somit mit dem Szintillator 22 verklebt.

Der Aufbau der einzelnen Detektormodule 55 ist insbesondere in Fig. 16 gut zu erkennen, deren Bildebene senkrecht zu der Bildebene der Fig. 15 steht. Die Detektormodule 55 umfassen jeweils ein eigenes Substrat 58, auf dem die Halbleiterdetektoren 9 und die zugehörigen Ausleseelektroniken 41 angeordnet sind. An der von dem Substrat abgewandten Seite des Halbleiterdetektors 9 ist die faseroptische Platte 12 angeordnet. Durch deren gekrümmte von dem Halbleiterdetektor abgewandte Vorderseite 21 und die schräg verlaufenden Seitenflächen 14, 15 können die faseroptischen Platten 12 der Kontur des Szintillators 22 im Wesentlichen ohne einen Spalt zwischen diesen untereinander bzw. zwischen diesen und dem Szintillator 22 folgen. Zugleich bieten sie eine ebene Auflagefläche für den Halbleiterdetektor 9.

Durch die beschriebenen Ansätze kann mit geringem technischem Aufwand eine großflächige gekrümmte Detektoroberfläche bereitgestellt werden, die senkrecht zur Detektoroberfläche eine geringe Bauhöhe aufweist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgendetektor für eine Röntgeneinrichtung (1) **dadurch gekennzeichnet, dass** eine konvexe oder im Wesentlichen hohlzylindersegmentförmige Detektoroberfläche (8) des Röntgendetektors (6) gebildet wird, indem mehrere Halbleiterdetektoren (9, 10, 32, 39) genutzt werden, an deren der Detektoroberfläche (8) zugewandten Vorderseite (21) jeweils eine dem jeweiligen Halbleiterdetektor (9, 10) zugeordnete faseroptische Platte (12, 13) angeordnet ist, wobei die faseroptischen Platten (12, 13) jeweils wenigstens eine Seitenfläche (14, 15, 16, 17), die einen spitzen Winkel (18) mit der Vorderseite (11) des zugeordneten Halbleiterdetektors (9, 10) einschließt und die mit einer Seitenfläche (14, 15, 16, 17) einer weiteren der faseroptischen Platten (12, 13) verbunden ist, und/oder eine gekrümmte von dem Halbleiterdetektor (9, 10) abgewandte Vorderseite (21) aufweisen, oder indem wenigstens ein Halbleiterdetektor (32, 39) des Röntgendetektors (3) durch ein Substrat (33) getragen wird, dessen dem Halbleiterdetektor (32, 39) zugewandte Seite (34) und dessen von dem Halbleiterdetektor (32, 39) abgewandte Seite (35) gekrümmt sind.

2. Röntgendetektor nach Anspruch 1, **dadurch gekennzeichnet, dass** die dem Halbleiterdetektor (9, 10) zugewandte Rückseite (20) der faseroptischen Platten (12, 13) jeweils eben ist.

3. Röntgendetektor nach Anspruch 2, **dadurch gekennzeichnet, dass** wenigstens eine Seitenfläche (14, 15, 16, 17) wenigstens einer der faseroptischen Platten (12, 13) senkrecht auf der Vorderseite (21) dieser faseroptischen Platte (12, 13) steht und/oder dass die Vorderseite (21) der faseroptischen Platte (12, 13) wenigstens in einer senkrecht auf der Vorderseite (11) des Halbleiterdetektors (9, 10) stehenden Schnittebene eine Kreissegmentform aufweist, wobei sich die Seitenfläche (14, 15, 16, 17) in Radialrichtung dieses Kreissegments erstreckt.

4. Röntgendetektor nach einem der vorangehenden Ansprüche, **dadurch gekennzeichnet, dass** wenigstens eine faseroptische Platte (12, 13) die Vorderseite (11) des zugeordneten Halbleiterdetektors (9, 10) oder dessen fotoaktive Fläche zumindest in eine Richtung vollständig bedeckt oder zumindest an einer oder zumindest an zwei Seiten über den Halbleiterdetektor (9, 10) oder dessen fotoaktive Fläche hinausragt.

5. Röntgendetektor nach einem der vorangehenden Ansprüche, **dadurch gekennzeichnet, dass** die Fasern (26) der jeweiligen faseroptischen Platte (9, 10) senkrecht zu der Vorderseite (11) des zugeordneten Halbleiterdetektors (9, 10) stehen oder dass ein Winkel (48) zwischen den Fasern (26) der faseroptischen Platte (12, 13) und der Vorderseite (11) des Halbleiterdetektors (9, 10) in wenigstens einer Richtung monoton mit der Position des halbleiterdetektorseitigen Endes (31) der Fasern (26) variiert.

6. Röntgendetektor nach einem der vorangehenden Ansprüche, **dadurch gekennzeichnet, dass** auf die von dem Halbleiterdetektor (9, 10) abgewandte Vorderseite (21) der jeweiligen faseroptischen Platten (12, 13) oder auf eine durch die Vorderseiten (21) von mehreren über ihre jeweiligen Seitenflächen (14, 15, 16, 17) verbundenen faseroptischen Platten (12, 13) gebildete Gesamtvorderseite ein Szintillator (22) aufgebracht ist.

7. Röntgendetektor nach einem der vorangehenden Ansprüche, **dadurch gekennzeichnet, dass** der oder ein auf die faseroptische Platte (12, 13, 36) oder auf die Gesamtvorderseite oder auf den Halbleiterdetektor (9, 10, 32, 39) aufgebrachter Szintillator (22, 37) als separate biegbare oder gekrümmte Komponente ausgebildet ist, die auf den Halbleiterdetektor (9, 10, 32, 39) oder die faseroptische Platte oder die Gesamtvorderseite aufgebracht, insbesondere aufgeklebt, ist..

8. Röntgendetektor nach einem der vorangehenden Ansprüche, **dadurch gekennzeichnet, dass** das Substrat (33) aus Glas oder aus kristallinem Silizium oder aus einem Polymermaterial oder aus einer Keramik oder aus einem faserverstärkten Material besteht oder wenigstens eines dieser Materialien umfasst.

9. Röntgendetektor nach einem de vorangehenden Ansprüche, **dadurch gekennzeichnet, dass** der Röntgendetektor (6) eine Detektorfläche (8) von wenigstens 400 cm² aufweist.

10. Röntgendetektor nach einem der vorangehenden Ansprüche, **dadurch gekennzeichnet, dass** das Substrat (33) mehrere Leitungen (44) ausbildet oder mehrere Leitungen (44) durch das Substrat (33) geführt sind, über die der Halbleiterdetektor (32, 39) kontaktiert ist.

11. Röntgendetektor nach einem der vorangehenden Ansprüche, **dadurch gekennzeichnet, dass** zwischen dem Halbleiterdetektor (9, 10, 32, 39) und der faseroptischen Platte (12, 13, 36) und/oder zwischen der faseroptischen Platte (12, 13, 26) und dem oder einem Szintillator (22, 37) und/oder zwischen dem Halbleiterdetektor und dem oder einem Szintillator (22, 37) eine zur Verminderung oder Reduzierung von Reflexionen an dem jeweiligen Materialübergang und insbesondere zur Verklebung dieser Komponenten dienende Kopplungsschicht (57) angeordnet ist.

12. Röntgeneinrichtung mit einer Röntgenquelle (49), **dadurch gekennzeichnet, dass** sie einen Röntgendetektor (6) nach einem der vorangehenden Ansprüche umfasst.

13. Röntgeneinrichtung nach Anspruch 12, **dadurch gekennzeichnet, dass** die Röntgeneinrichtung zusätzlich eine Magnetresonanzeinrichtung zur Erfassung von Magnetresonanzdaten umfasst, wobei der Röntgendetektor innerhalb eines von dem Hauptmagneten (50) der Magnetresonanzeinrichtung umgriffenen, insbesondere zylinderförmigen, Hauptmagnetvolumen angeordnet ist.

14. Röntgeneinrichtung nach Anspruch 13, **dadurch gekennzeichnet, dass** die Magnetresonanzeinrichtung wenigstens eine Körperspule zum senden und/oder empfangen von Hochfrequenzsignalen, die innerhalb des Hauptmagnetvolumens angeordnet ist und einen insbesondere zylinderförmigen Untersuchungsbereich umgreift, und eine Gradientenspule zur Erzeugung von Gradientenfeldern, die zwischen der Körperspule und dem Hauptmagneten (50) angeordnet ist, umfasst, wobei der Röntgendetektor zwischen der Gradientenspule und der Körperspule angeordnet ist.

15. Röntgeneinrichtung nach einem der Ansprüche 13 oder 14, **dadurch gekennzeichnet, dass** eine Ausleseelektronik (41) zum Auslesen des Halbleiterdetektors (8, 9, 32, 39) in Axialrichtung des Hauptmagnetvolumens oder des Untersuchungsvolumens versetzt neben dem Halbleiterdetektor (8, 9, 32, 39) angeordnet ist.

16. Röntgeneinrichtung nach einem der Ansprüche 12 bis 15, **dadurch gekennzeichnet, dass** die oder eine Ausleseelektronik (41) zum Auslesen des Halbleiterdetektors (8, 9, 32, 39) auf einem gekrümmten Schaltungsträger angeordnet ist.

17. Röntgeneinrichtung nach einem der Ansprüche 12 bis 16, **dadurch gekennzeichnet, dass** in wenigstens einer senkrecht auf der Vorderseite (21) des Halbleiterdetektors stehenden Schnittebene die in dieser Schnittebene liegenden Fasern (26) der faseroptischen Platte (12, 13) in Radialrichtung bezüglich eines Fokuspunktes (51) der Röntgenquelle (49) oder bezüglich einer Geraden, die diesen Fokuspunkt (51) umfasst, ausgerichtet sind.

18. Röntgeneinrichtung nach einem der Ansprüche 12 bis 17, **dadurch gekennzeichnet, dass** sie eine Verarbeitungseinrichtung (59) zur Verarbeitung von durch den Röntgendetektor (6) erfassten Rohbilddaten umfasst, wobei die Verarbeitungseinrichtung (59) dazu eingerichtet ist, die Rohbilddaten oder ein durch eine Vorverarbeitung aus den Rohbilddaten ermitteltes Zwischenbild in zwei, insbesondere orthogonale, Richtungen unterschiedlich stark zu dehnen oder zu stauchen oder in eine dieser Richtungen zu dehnen und in die andere zu stauchen, um eine unterschiedlich starke Krümmung des Röntgendetektors in die zwei Richtungen zu kompensieren.

19. Röntgeneinrichtung nach einem der Ansprüche 12 bis 18, **dadurch gekennzeichnet, dass** der Halbleiterdetektor (9, 10, 32, 38) derart an der faseroptischen Platte (12, 13, 36) angeordnet ist, dass erste Fasern der faseroptischen Platte von einem Bildgebungsbereich (52) des Halbleiterdetektors (9, 10, 32, 38) zu der von dem Halbleiterdetektor (9, 10, 32, 38) abgewandten Vorderseite (21) der faseroptischen Platte (12, 13, 36) geführt sind und dass zweite Fasern der faseroptischen Platte (12, 13, 36) von einem Referenzbereich (53) des Halbleiterdetektors (9, 10, 32, 38) zu der oder einer weiteren Seitenfläche (14, 15, 16, 17) der faseroptischen Platte (12, 13, 36) geführt sind, wobei die Röntgeneinrichtung (1) die oder eine Verarbeitungseinrichtung (59) umfasst, die dazu eingerichtet ist, Ergebnisbilddaten aus durch den Röntgendetektor (6) erfassten Rohbilddaten zu ermitteln, wobei die Bilddaten wenigstens eines Bildpunktes der Ergebnisbilddaten sowohl von Rohbilddaten abhängen, die im Bildgebungsbereich (52) des Halbleiterdetektors erfasst werden, als auch von Rohbilddaten, die in dem Referenzbereich (53) des Halbleiterdetektors erfasst werden.

20. Verfahren zur Herstellung eines Röntgendetektors mit einer konvexen oder im Wesentlichen hohlzylindersegmentförmigen Detektoroberfläche (8), indem
- entweder mehrere Halbleiterdetektoren bereitgestellt werden, deren der Detektoroberfläche (8) zugewandte Vorderseite (21) jeweils an einer dem jeweiligen Halbleiterdetektor (9, 10) zugeordneten faseroptische Platte (12, 13) angebracht wird, wobei faseroptische Platten (12, 13) verwendet werden, die eine gekrümmte von dem Halbleiterdetektor (9, 10) abgewandte Vorderseite (21) aufweisen und/oder die jeweils wenigstens eine Seitenfläche (14, 15, 16, 17) aufweisen, die nach dem Anbringen der faseroptischen Platte (12, 13) an der Vorderseite (11) des zugeordneten Halbleiterdetektors (9, 10) einen spitzen Winkel (18) mit der Vorderseite (11) einschließt, wobei diese Seitenfläche mit einer Seitenfläche (14, 15, 16, 17) einer weiteren der faseroptischen Platten (12, 13) verbunden wird,
- oder indem ein bereitgestellter Halbleiterdetektor (32, 39) gekrümmt und an einer gekrümmten Halteeinrichtung (46) angebracht wird, wonach der Halbleiterdetektor (32, 39) mit Kontakten (43, 47) eines Substrats (33), dessen dem Halbleiterdetektor (32, 39) zugewandte Seite (34) und von dem Halbleiterdetektor (32, 39) abgewandte Seite (35) gekrümmt sind, elektrisch kontaktiert wird, wonach der Halbleiterdetektor (32, 39) von der Halteeinrichtung (46) abgelöst wird.
